# EUROPÄISCHE PATENTANMELDUNG

(11) **EP 2 025 673 A1**
(43) Veröffentlichungstag der Anmeldung: **18.02.2009**
(21) Anmeldenummer: 08102043.0
(22) Anmeldetag: 26.02.2008
(51) Int. Cl.: C07D 243/08, C07D 295/14, C07C 311/29

(54) **Arylsulfonamide mit analgetischer Wirkung**

(30) Priorität: 14.08.2007 WO PCT/EP2007/058408; 21.02.2008 WO PCT/EP2008/052157
(71) Anmelder: Boehringer Ingelheim International GmbH, 55216 Ingelheim am Rhein (DE)
(72) Erfinder: Die Erfindernennung liegt noch nicht vor
(74) Vertreter: Hammann, Heinz

(57) **Zusammenfassung**

Gegenstand der vorliegenden Erfindung Verbindungen der allgemeinen Formel **I** 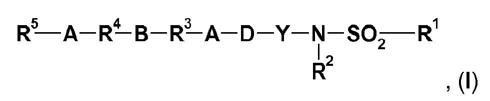 in der **A, B, D, Y, R**¹, **R**², **R**³, **R**⁴ und **R**⁵ wie in der Beschreibung erwähnt definiert sind, deren Enantiomere, deren Diastereomere, deren Gemische und deren Salze, insbesondere deren physiologisch verträgliche Salze mit organischen oder anorganischen Säuren oder Basen, welche wertvolle Eigenschaften aufweisen, deren Herstellung, die die pharmakologisch wirksamen Verbindungen enthaltenden Arzneimittel, deren Herstellung und deren Verwendung.

## Beschreibung

### Gegenstand der vorliegenden Erfindung sind Verbindungen der allgemeinen Formel I

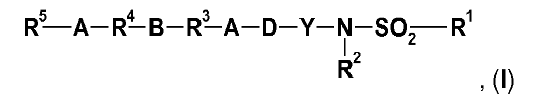

in der **A, B, D, Y, R¹, R², R³, R⁴** und **R⁵** wie nachstehend erwähnt definiert sind, deren Enantiomere, deren Diastereomere, deren Gemische und deren Salze, insbesondere deren physiologisch verträgliche Salze mit organischen oder anorganischen Säuren oder Basen, welche wertvolle Eigenschaften aufweisen, deren Herstellung, die die pharmakologisch wirksamen Verbindungen enthaltenden Arzneimittel, deren Herstellung und deren Verwendung.

### DETAILLIERTE BESCHREIBUNG DER ERFINDUNG

In der obigen allgemeinen Formel I bedeuten in einer ersten Ausführungsform
- **A**: eine Bindung,
- **B**: eine Bindung,
- **D-Y**: zusammen eine Gruppe ausgewählt aus

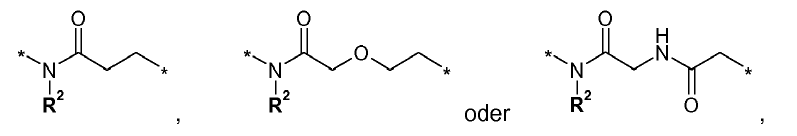

- **R¹**: die Gruppe

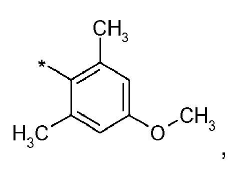

- **R²**: H oder C₁₋₃-Alkyl-, wobei jede Methylengruppe mit bis zu zwei und jede Methylgruppe mit bis zu drei Fluoratomen substituiert sein kann, oder auch H₃C-C(O)-,

- **R³**: eine C₄₋₆-Cycloalkylengruppe, die durch einen, zwei oder drei Reste **R^{3.1}** substituiert sein kann,
- **R^{3.1}**: -CH₃, -C₂H₅, *iso*-Propyl, *tert*-Butyl, -OH, F, Cl, Br, I,
- **R⁴**: einen gesättigten 6- oder 7-gliedrigen Diaza-Heterocyclus,
- **R⁵**: C₁₋₃-Alkyl- oder C₃₋₅-Cycloalkyl bedeutet,
deren Enantiomere, deren Diastereomere, deren Gemische und deren Salze, insbesondere deren physiologisch verträgliche Salze mit organischen oder anorganischen Säuren oder Basen.

Eine zweite Ausführungsform der vorliegenden Erfindung besteht in den Verbindungen der obigen allgemeinen Formel **I**, in denen
- **A**: eine Bindung,
- **B**: eine Bindung,
- **D-Y**: zusammen eine Gruppe ausgewählt aus

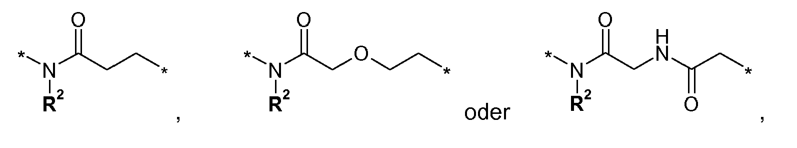

- **R¹**: die Gruppe

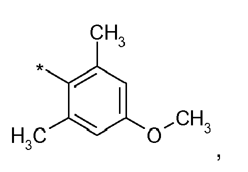

- **R²**: H oder C₁₋₃-Alkyl-, wobei jede Methylengruppe mit bis zu zwei und jede Methylgruppe mit bis zu drei Fluoratomen substituiert sein kann, oder auch H₃C-C(O)-,

- **R**³: eine C₄₋₆-Cycloalkylengruppe,
- **R⁴**: einen gesättigten 6- oder 7-gliedrigen Diaza-Heterocyclus,
- **R⁵**: C₁₋₃-Alkyl- oder C₃₋₅-Cycloalkyl bedeutet,
deren Enantiomere, deren Diastereomere, deren Gemische und deren Salze, insbesondere deren physiologisch verträgliche Salze mit organischen oder anorganischen Säuren oder Basen.

Eine dritte Ausführungsform der vorliegenden Erfindung besteht in den Verbindungen der obigen allgemeinen Formel **I**, in denen **A, B, D, Y, R¹, R², R⁴** und **R⁵** wie voranstehend unter der ersten Ausführungsform erwähnt definiert sind und
- **R³**: eine C₄₋₆-Cycloalkylengruppe, die durch einen, zwei oder drei Reste **R^{3.1}** substituiert sein kann, und
- **R^{3.1}**: -CH₃, -C₂H₅, *iso*-Propyl, *tert*-Butyl, -OH, F, Cl, Br oder I bedeutet,
mit der Maßgabe, dass die voranstehend erwähnte C₄₋₆-Cycloalkylengruppe in 1,3-Stellung mit dem restlichen Molekül verknpüft ist, deren Enantiomere, deren Diastereomere, deren Gemische und deren Salze, insbesondere deren physiologisch verträgliche Salze mit organischen oder anorganischen Säuren oder Basen.

Eine vierte Ausführungsform der vorliegenden Erfindung besteht in den Verbindungen der obigen allgemeinen Formel **I,** in denen **A, B, D, Y, R¹, R², R⁴** und **R⁵** wie voranstehend unter der zweiten Ausführungsform erwähnt definiert sind und
- **R³**: eine C₄₋₆-Cycloalkylengruppe, die durch einen, zwei oder drei Reste **R^{3.1}** substituiert sein kann, und

- **R^{3.1}**: -CH₃, -C₂H₅, *iso*-Propyl, *tert*-Butyl, -OH, F, Cl, Br oder I bedeutet,
mit der Maßgabe, dass die voranstehend erwähnte C₄₋₆-Cycloalkylengruppe in 1,3-Stellung mit dem restlichen Molekül verknpüft ist, deren Enantiomere, deren Diastereomere, deren Gemische und deren Salze, insbesondere deren physiologisch verträgliche Salze mit organischen oder anorganischen Säuren oder Basen.

Als besonders bevorzugte Verbindungen der obigen allgemeinen Formel **I** seien beispielsweise folgende genannt:

| **Nr.** | **Struktur** |
|---|---|
| (1) | 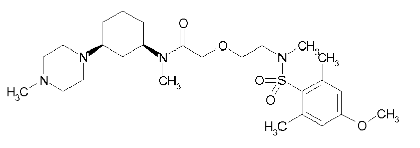 |
| (2) | 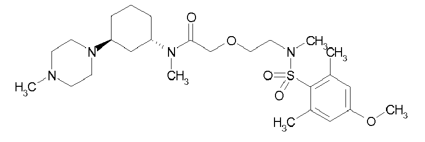 |
| (3) | 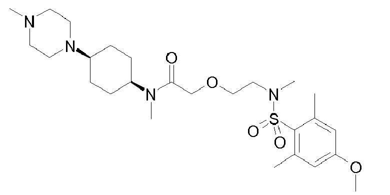 |
| (4) | 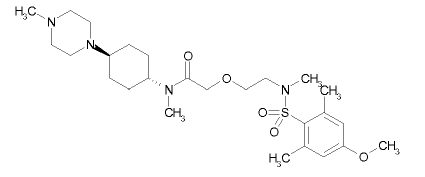 |
| (5) | 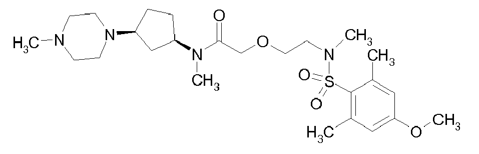 |
| (6) | 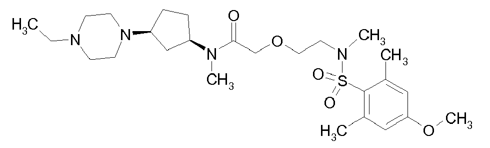 |
| (7) | 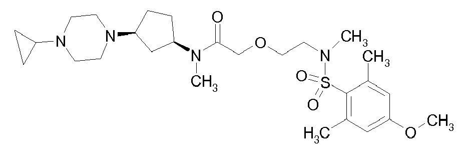 |
| (8) | 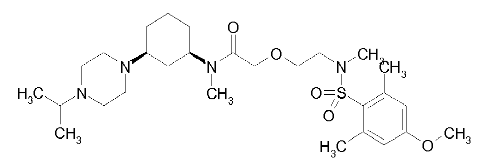 |
| (9) | 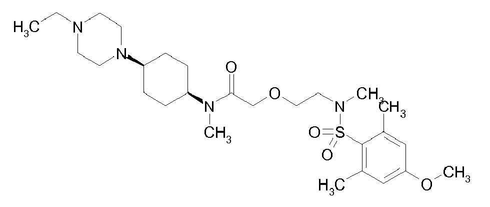 |
| (10) | 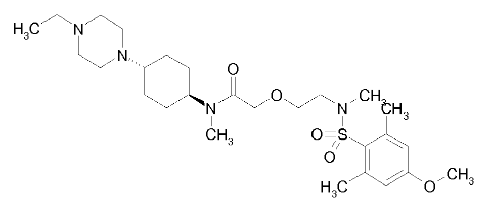 |
| (11) | 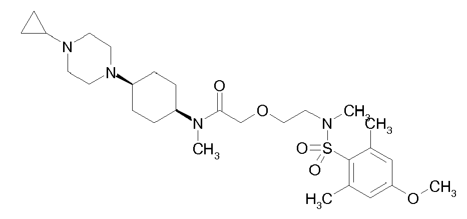 |
| (12) | 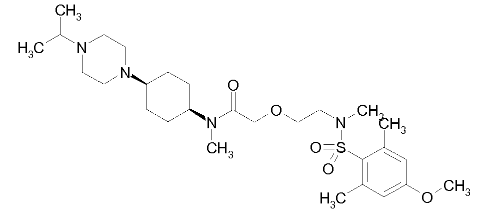 |
| (13) | 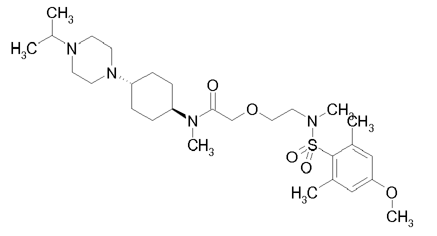 |
| (14) | 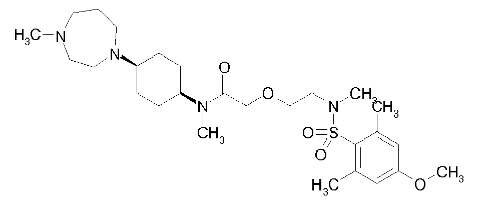 |
| (15) | 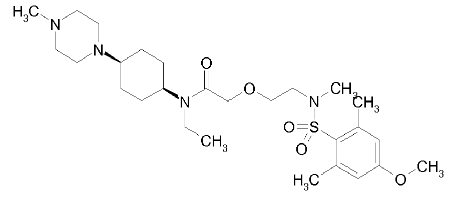 |
| (16) | 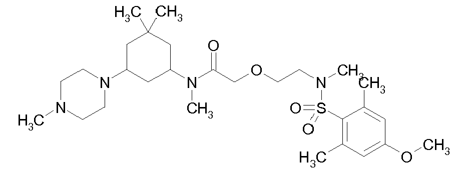 |
| (17) | 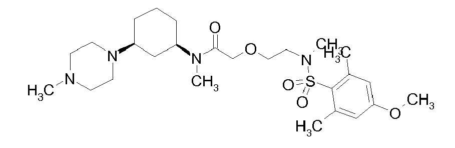 |
| (18) | 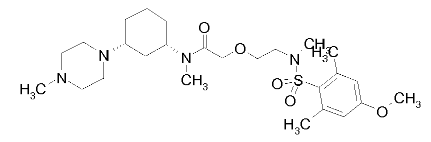 |
| (19) | 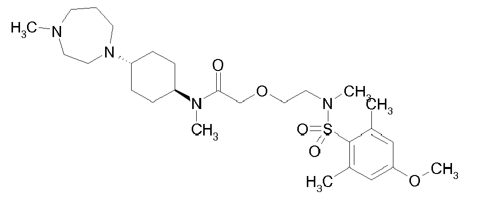 |
| (20) | 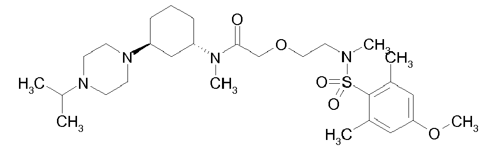 |
| (21) | 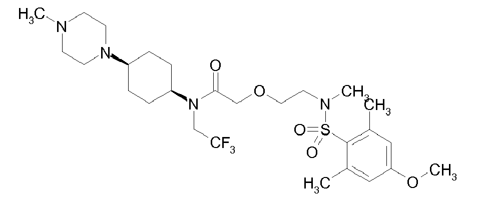 |
| (22) | 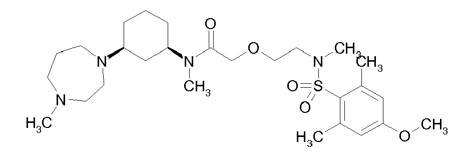 |
| (23) | 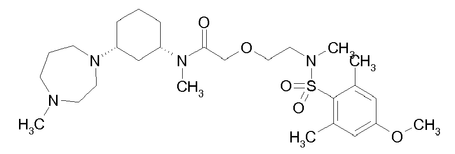 |
| (24) | 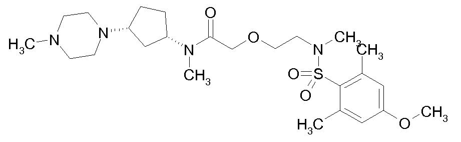 |
| (25) | 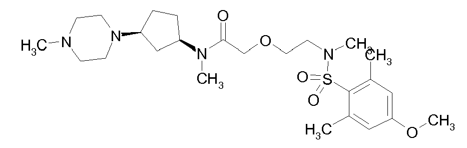 |
| (26) | 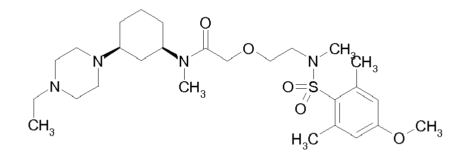 |
| (27) | 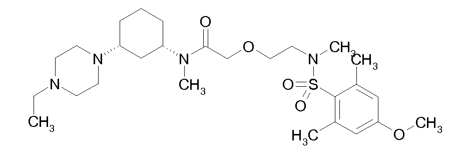 |
| (28) | 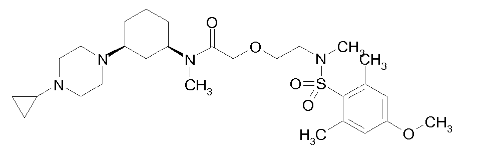 |
| (29) | 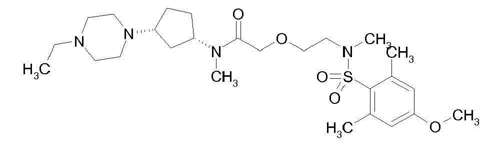 |
| (30) | 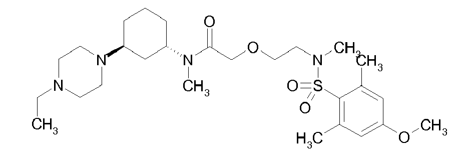 |
| (31) | 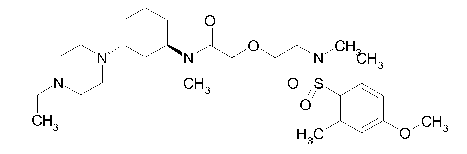 |
| (32) | 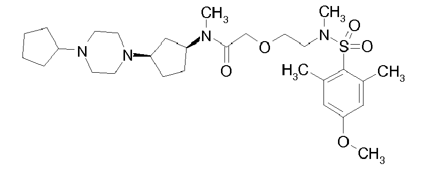 |
| (33) | 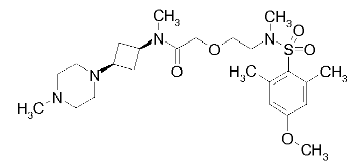 |

deren Enantiomere, deren Diastereomere, deren Gemische und deren Salze, insbesondere deren physiologisch verträgliche Salze mit organischen oder anorganischen Säuren oder Basen.

Als ganz besonders bevorzugte Verbindungen der obigen allgemeinen Formel **I** seien beispielsweise folgende genannt:

| **Nr.** | **Struktur** |
|---|---|
| (1) | 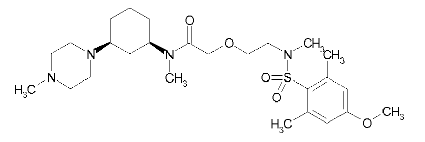 |
| (2) | 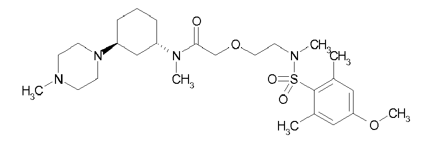 |
| (3) | 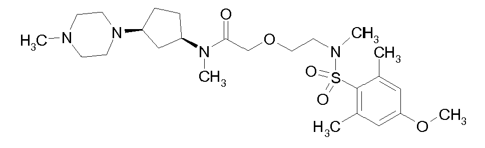 |
| (4) | 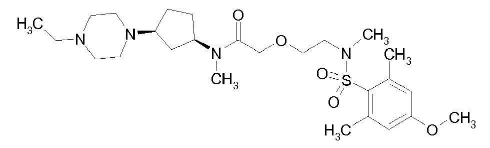 |
| (5) | 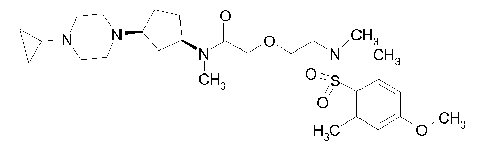 |
| (6) | 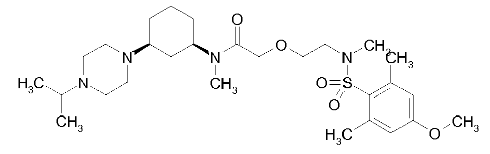 |
| (7) | 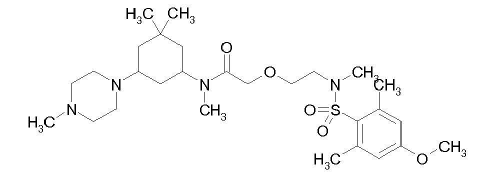 |
| (8) | 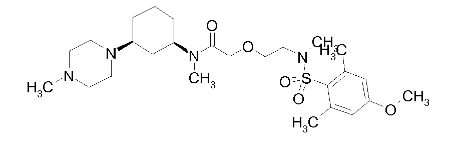 |
| (9) | 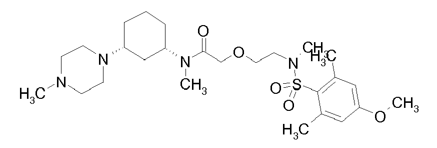 |
| (10) | 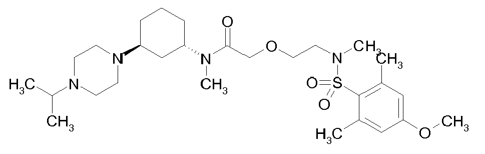 |
| (11) | 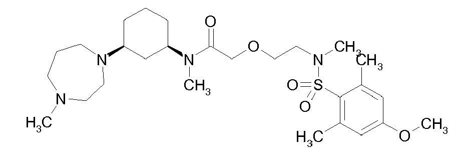 |
| (12) | 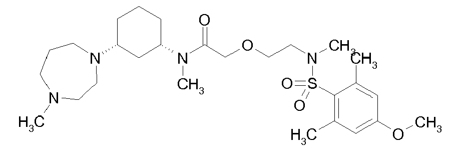 |
| (13) | 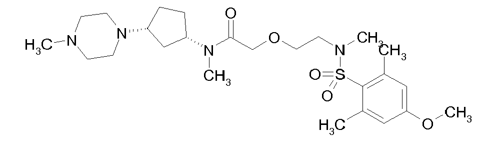 |
| (14) | 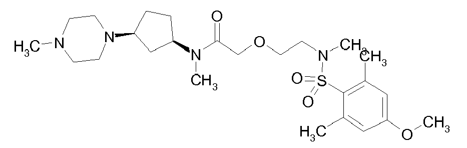 |
| (15) | 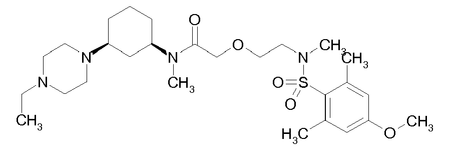 |
| (16) | 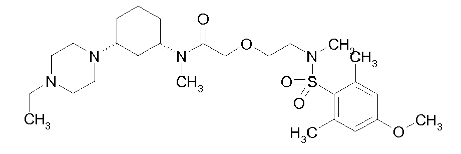 |
| (17) | 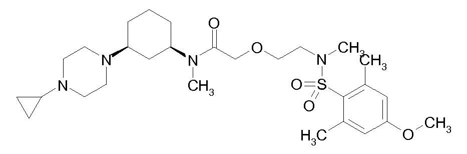 |
| (18) | 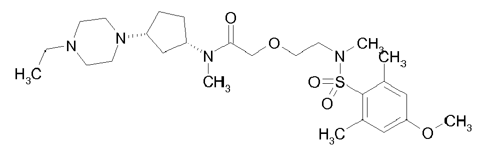 |
| (19) | 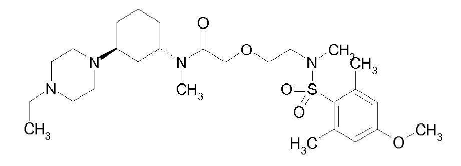 |
| (20) | 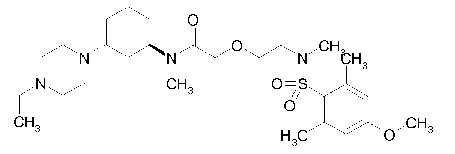 |
| (21) | 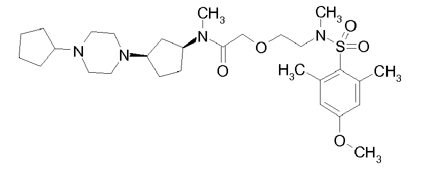 |
| (22) | 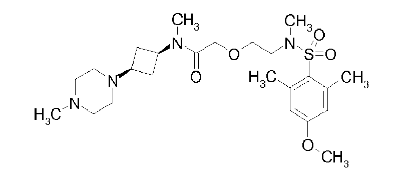 |

deren Enantiomere, deren Diastereomere, deren Gemische und deren Salze, insbesondere deren physiologisch verträgliche Salze mit organischen oder anorganischen Säuren oder Basen.

### VERWENDETE BEGRIFFE UND DEFINITIONEN

Soweit nicht anders angegeben, sind alle Substituenten voneinander unabhängig. Sollten an einer Gruppe z.B. mehrere C₁₋₆-Alkylgruppen als Substituenten sein, so könnte im Fall von drei Substituenten C₁₋₆-Alkyl unabhängig voneinander einmal Methyl, einmal *n*-Propyl und einmal *tert*-Butyl bedeuten.

Im Rahmen dieser Anmeldung können bei der Definition von möglichen Substituenten, diese auch in Form einer Strukturformel dargestellt werden. Dabei wird, falls vorhanden, ein Stern (*) in der Strukturformel des Substituenten als der Verknüpfungspunkt zum Rest des Moleküls verstanden.

Ebenfalls mit vom Gegenstand dieser Erfindung umfasst sind die erfindungsgemäßen Verbindungen, einschließlich deren Salze, in denen ein oder mehrere Wasserstoffatome, beispielsweise ein, zwei, drei, vier oder fünf Wasserstoffatome, durch Deuterium ausgetauscht sind.

Unter dem Begriff "C₁₋₃-Alkyl" (auch soweit sie Bestandteil anderer Reste sind) werden Alkylgruppen mit 1 bis 3 Kohlenstoffatomen verstanden. Beispielsweise werden hierfür genannt: Methyl, Ethyl, *n*-Propyl und *iso*-Propyl. Gegebenenfalls werden für die vorstehend genannten Gruppen auch die Abkürzungen Me, Et, n-Pr, *i*-Pr etc. verwendet. Sofern nicht anders beschrieben, umfaßt die Definition Propyl alle denkbaren isomeren Formen des Restes. So umfasst Propyl *n*-Propyl und *iso*-Propyl.
Weiterhin umfassen die voranstehend genannten Begriffe auch solche Reste, in denen jede Methylengruppe mit bis zu zwei und jede Methylgruppe mit bis zu drei Fluoratomen substituiert sein kann.

Unter dem Begriff "C₃₋₅-Cycloalkyl" (auch soweit sie Bestandteil anderer Reste sind) werden cyclische Alkylgruppen mit 3 bis 5 Kohlenstoffatomen verstanden. Beispielsweise werden hierfür genannt: Cyclopropyl, Cyclobutyl oder Cyclopentyl. Soweit nicht anders beschrieben, können die cyclischen Alkylgruppen substituiert sein mit einem oder mehreren Resten ausgewählt aus der Gruppe bestehend aus Methyl, Ethyl, *iso*-Propyl, *tert*-Butyl, Hydroxy, Fluor, Chlor, Brom und Jod.

Unter dem Begriff "C₄₋₆-Cycloalkylen" (auch soweit sie Bestandteil anderer Reste sind) werden cyclische Alkylengruppen mit 4 bis 6 Kohlenstoffatomen verstanden. Beispielsweise werden hierfür genannt: Cyclobutylen, Cyclopentylen oder Cyclohexylen. Soweit nicht anders beschrieben, können die cyclischen Alkylengruppen substituiert sein mit einem oder mehreren Resten ausgewählt aus der Gruppe bestehend aus Methyl, Ethyl, *iso*-Propyl, *tert*-Butyl, Hydroxy, Fluor, Chlor, Brom und Jod.
Eine C₄₋ oder eine C₅-Cycloalkylengruppe kann in 1,2-Stellung oder in 1,3-Stellung mit dem restlichen Molekül verknpüft sein, vorzugsweise in 1,3-Stellung. Eine C₆-Cycloalkylengruppe kann in 1,2-Stellung, in 1,3- Stellung oder in 1,4-Stellung mit dem restlichen Molekül verknüpft sein, vorzugsweise in 1,3-Stellung.

Unter dem Begriff "gesättigte Diaza-Heterocyclen" werden sechs- oder siebengliedrige heterocyclische Ringe verstanden, die zwei Stickstoffatome enthalten. Dabei ist der Ring über beide Stickstoffatome mit dem restlichen Molekül verknüpft. Als Beispiele werden genannt:

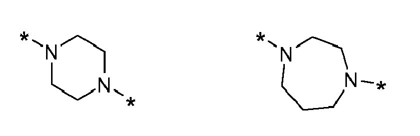

Verbindungen der allgemeinen Formel **I** können, sofern sie geeignete basische Funktionen enthalten, beispielsweise Aminogruppen, insbesondere für pharmazeutische Anwendungen in ihre physiologisch verträglichen Salze mit anorganischen oder organischen Säuren übergeführt werden. Als anorganische Säuren kommen hierfür beispielsweise Bromwasserstoffsäure, Phosphorsäure, Salpetersäure, Salzsäure, Schwefelsäure, Methansulfonsäure, Ethansulfonsäure, Benzolsulfonsäure oder p-Toluolsulfonsäure in Frage, als organische Säuren kommen beispielsweise Äpfelsäure, Bernsteinsäure, Essigsäure, Fumarsäure, Maleinsäure, Mandelsäure, Milchsäure, Weinsäure oder Zitronensäure in Betracht. Weiterhin können gegebenenfalls im Molekül vorhandene tertiäre Aminogruppen quarternisiert werden. Zur Umsetzung werden dabei Alkylhalogenide eingesetzt. Erfindungsgemäß bevorzugt wird für die Quartenisierung Methyliodid verwendet.

Weiterhin lassen sich die Verbindungen der allgemeinen Formel **I**, sofern sie geeignete Carbonsäurefunktionen enthalten, gewünschtenfalls in ihre Additionssalze mit anorganischen oder organischen Basen überführen. Als anorganische Basen kommen hierfür beispielsweise Alkali- oder Erdalkalimetallhydroxide, beispielsweise Natriumhydroxid oder Kaliumhydroxid, oder Carbonate, Ammoniak, Zink- oder Ammoniumhydroxide in Frage; als organische Amine kommen beispielsweise Diethylamin, Triethylamin, Ethanolamin, Diethanolamin, Triethanolamin, Cyclohexylamin oder Dicyclohexylamin in Betracht.

Die erfindungsgemäßen Verbindungen können als Racemate vorliegen, sofern sie nur ein Chiralitätselement besitzen, sie können aber auch als reine Enantiomere, d.h. in (R)- oder (S)-Form gewonnen werden.

Die Anmeldung umfasst jedoch auch die einzelnen diastereomeren Antipodenpaare oder deren Gemische, die dann vorliegen, wenn mehr als ein Chiralitätselement in den Verbindungen der allgemeinen Formel **I** vorhanden ist, sowie die einzelnen optisch aktiven Enatiomeren, aus denen sich die erwähnten Racemate zusammensetzen.

Gegenstand der Erfindung sind die jeweiligen Verbindungen gegebenenfalls in Form der einzelnen optischen Isomeren, Mischungen der einzelnen Enantiomeren oder Racemate, in Form der Tautomere sowie in Form der freien Basen oder der entsprechenden Säureadditionssalze mit pharmakologisch unbedenklichen Säuren - wie beispielsweise Säureadditionssalze mit Halogenwasserstoffsäuren - beispielsweise Chlor- oder Bromwasserstoffsäure - oder organische Säuren - wie beispielsweise Oxal-, Fumar-, Diglycol- oder Methansulfonsäure.

### HERSTELLVERFAHREN

Erfindungsgemäß erhält man die Verbindungen der allgemeinen Formel **I** nach an sich bekannten Verfahren, beispielsweise nach folgenden Verfahren:

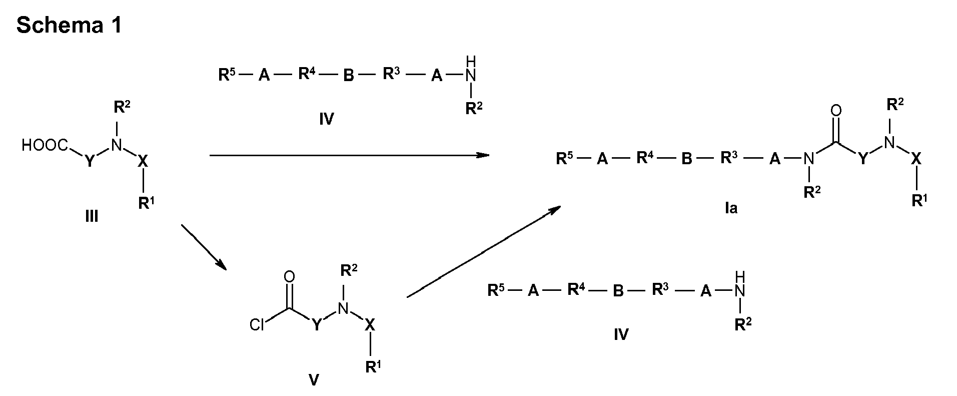

Die in Schema 1 dargestellte Verknüpfung von Carbonsäuren der allgemeinen Formel **III**, in der alle Reste wie voranstehend erwähnt definiert sind, mit Aminen der allgemeinen Formel **IV**, in der alle Reste wie voranstehend erwähnt definiert sind, unter Bildung von Carbonsäureamiden der allgemeinen Formel **Ia**, in der alle Reste wie voranstehend erwähnt definiert sind, kann mit herkömmlichen Methoden zur Amidbildung durchgeführt werden.

Die Kupplung wird bevorzugt unter Verwendung von aus der Peptidchemie bekannten Verfahren (siehe z. B. Houben-Weyl, Methoden der Organischen Chemie, Bd. 15/2) durchgeführt, wobei zum Beispiel Carbodiimide, wie z.B. Dicyclohexylcarbodiimid (DCC), Diisopropylcarbodiimid (DIC) oder Ethyl-(3-dimethylamino-propyl)-carbodiimid, *O*-(1*H*-Benzotriazol-1-yl)-*N*,*N*-*N*',*N*'-tetramethyluronium-hexafluorphosphat (HBTU) oder
- tetrafluorborat (TBTU) oder 1*H*-Benzotriazol-1-yl-oxy-tris-(dimethylamino)-phosphonium-hexafluorphosphat (BOP) eingesetzt werden. Durch Zugabe von 1-Hydroxybenzotriazol (HOBt) oder von 3-Hydroxy-4-oxo-3,4-di-hydro-1,2,3-benzotriazin (HOObt) kann die Reaktionsgeschwindigkeit gesteigert werden. Die Kupplungen werden normalerweise mit äquimolaren Anteilen der Kupplungskomponenten sowie des Kupplungsreagenz in Lösemitteln wie Dichlormethan, Tetrahydrofuran (THF), Acetonitril, Dimethylformamid (DMF), Dimethylacetamid (DMA), N-Methylpyrrolidon (NMP) oder Gemischen aus diesen und bei Temperaturen zwischen -30°C und +30°C, bevorzugt -20°C und +25°C, durchgeführt. Sofern erforderlich, wird als zusätzliche Hilfsbase Diisopropylethylamin (DIPEA) (Hünig-Base) bevorzugt.

Eine alternative Methode zur Verknüpfung besteht in der Überführung einer Carbonsäure der allgemeinen Formel **III,** in der alle Reste wie voranstehend erwähnt definiert sind, in ein Carbonsäurechlorid der allgemeinen Formel **V**, in der alle Reste wie voranstehend erwähnt definiert sind, und anschließender Umsetzung mit einem Amin der allgemeinen Formel **IV,** in der alle Reste wie voranstehend erwähnt definiert sind. Die Synthese eines Carbonsäurechlorids der allgemeinen Formel **V** erfolgt nach literaturbekannten Verfahren (siehe z. B. Houben-Weyl, Methoden der Organischen Chemie, Bd. E5/1).

Die als Ausgangsstoffe verwendeten Carbonsäuren der allgemeinen Formel **III,** in der alle Reste wie voranstehend erwähnt definiert sind, erhält man nach an sich literaturbekannten Verfahren, beispielsweise durch die in Schema 2 bis 7 dargestellten Synthesewege.

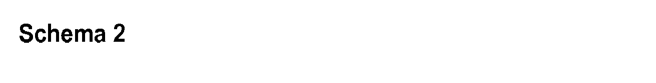

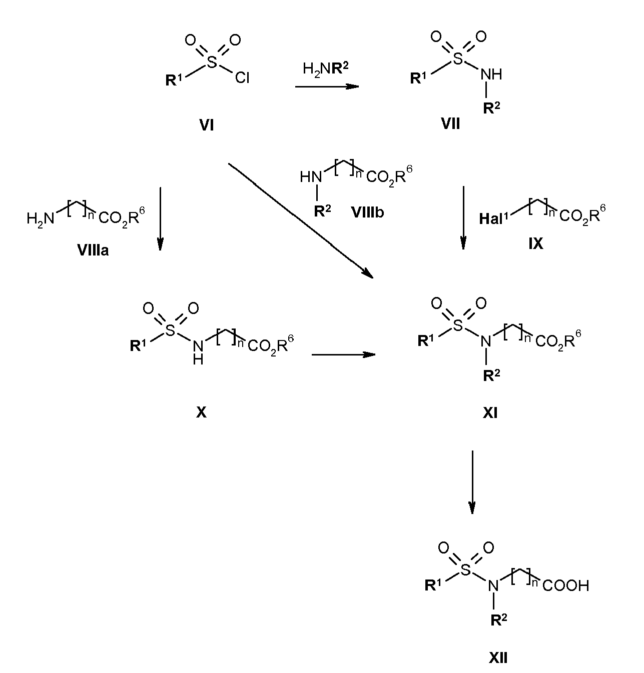

Die Sulfonsäurechloride der allgemeinen Formel **VI,** in der **R¹** wie voranstehend erwähnt definiert ist, sind entweder literaturbekannt oder käuflich erwerbbar. Sie werden nach Standard-Reaktionsbedingungen mit einem Amin der allgemeinen Formeln H₂N-**R²**, **VIIIa** oder **VIIIb** zu Sulfonsäureamiden der allgemeinen Formeln **VII, X** oder **XI** umgesetzt, wobei **R¹** und **R²** wie voranstehend erwähnt definiert sind und n eine Zahl 1, 2, 3 oder 4 und **R⁶** einen C₁₋₆-Alkylrest bedeuten. Die Umsetzung erfolgt gegebenenfalls in Gegenwart einer Base wie Triethylamin, DIPEA oder Pyridin und einem inerten Lösungsmittel wie Dichlormethan oder Tetrahydrofuran bei einer Temperatur von 0°C bis 100°C mit einer typischen Reaktionsdauer von einer bis 24 Stunden.

Die Umsetzung der Sulfonsäureamide der allgemeinen Formel **VII** mit einem Halogenid der allgemeinen Formel **IX**, in der **Hal¹** Chlor oder Brom bedeutet, erfolgt nach literaturbekannten Verfahren, beispielsweise mit Hilfe einer Base wie Kalium- oder Natriumcarbonat in Dimethylformamid oder Tetrahydrofuran bei 0°C bis 100°C.

Die Hydrolyse der Carbonsäurester der allgemeinen Formel **XI**, in der **R¹** und **R²** wie voranstehend erwähnt definiert sind, **n** eine Zahl 1, 2, 3 oder 4 und **R⁶** eine C₁₋₃-Alkylgruppe bedeuten, zu Carbonsäuren der allgemeinen Formel **XII,** in der **R¹** und **R²** wie voranstehend erwähnt definiert sind und n eine Zahl 1, 2, 3 oder 4 und **R⁶** eine C₁₋₃-Alkylgruppe bedeutet, wird unter bekannten Bedingungen durchgeführt, beispielsweise mit Lithium- oder Natriumcarbonat und Wasser in Methanol und/oder Tetrahydrofuran.

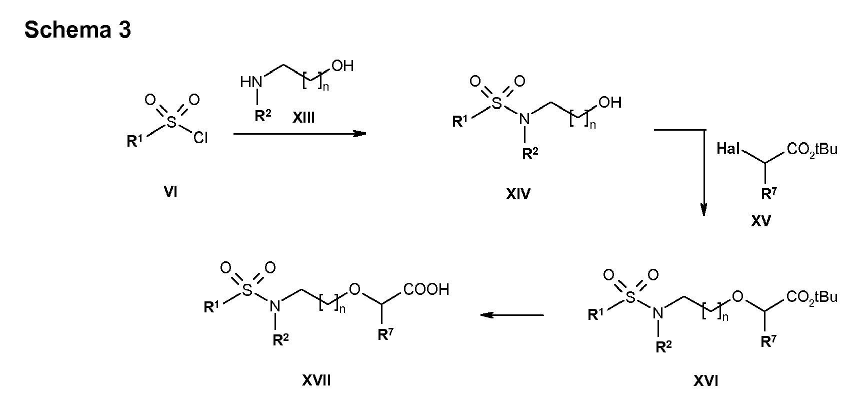

Die Herstellung von Sulfonsäureamiden der allgemeinen Formel **XIV** erfolgt wie unter Schema 2 beschrieben.

Die Alkylierung der Hydroxylfunktion der Sulfonsäureamide der allgemeinen Formel **XIV**, in der **R¹** und **R²** wie voranstehend erwähnt definiert sind mit der Maßgabe, dass **R²** kein Wasserstoffatom darstellt, und **n** eine Zahl 1, 2, 3 oder 4 und **R⁶** eine C₁₋₃-Alkylgruppe bedeutet, wird unter literaturbekannten Reaktionsbedingungen durchgeführt, beispielsweise unter 2-Phasen-Bedingungen mit Hilfe eines Phasentransfer-Katalysators in Gegenwart einer starken anorganischen Base wie Natronlauge oder Kalilauge und in einem inerten Lösungsmittel wie Toluol bei 0°C bis 100°C.

Die Spaltung des tert-Butylesters der allgemeinen Formel **XVI**, in der **R¹** und **R²** wie voranstehend erwähnt definiert sind, **n** eine Zahl 1, 2, 3 oder 4 und **R⁶** eine C₁₋₃-Alkylgruppe und **R⁷** ein Wasserstoffatom oder eine C₁₋₃-Alkylgruppe bedeuten, erfolgt nach literaturbekannten Verfahren (siehe z.B. Philip J. Kocieński, Protecting Groups, 3rd Edition, 2005, Georg Thieme Verlag).

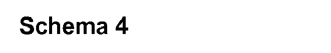

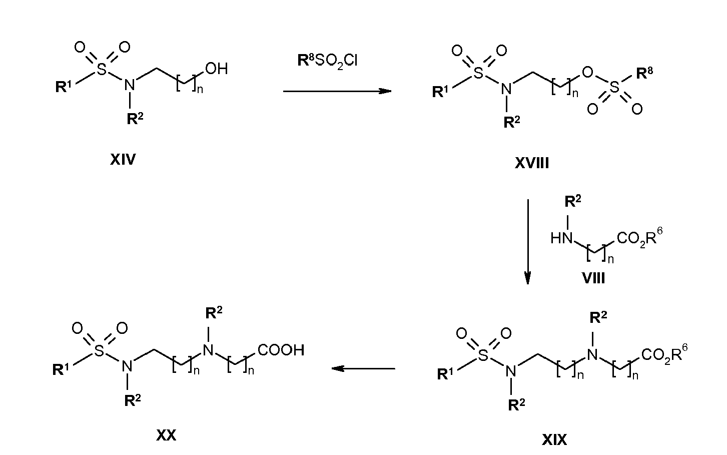

Die Sulfonierung der Hydroxylfunktion einer Verbindung der allgemeinen Formel **XIV,** in der **R¹** und **R²** wie voranstehend erwähnt definiert sind mit der Maßgabe, dass **R²** kein Wasserstoffatom darstellt, und **n** eine Zahl 1, 2, 3 oder 4 und **R⁶** eine C₁₋₃-Alkylgruppe bedeutet, mit einem Sulfonsäurechlorid der allgemeinen Formel **R⁸**SO₂Cl, in der **R⁸** eine C₁₋₃-Alkylgruppe oder eine ggf. durch C₁₋₃-Alkylgruppe substituierte Phenylgruppe darstellt, zu Verbindungen der allgemeinen Formel **XVIII,** in der alle Rest wie voranstehend erwähnt definiert sind, wird unter Standard-Reaktionsbedingungen durchgeführt, typischerweise in Gegenwart einer Base wie DMAP und/oder Pyridin und einem inerten Lösungsmittel wie Dichlormethan oder THF bei -5°C bis 35°C. Eine flüssige Base wie Pyridin kann als Base und gleichzeitig als Lösungsmittel verwendet werden.

Die nachfolgende Alkylierung der Amine mit der allgemeinen Formel **VII** zu Verbindungen der allgemeinen Formel **XIX,** in der **R¹** und **R²** wie voranstehend erwähnt definiert sind, **n** eine Zahl 1, 2, 3 oder 4 und **R⁶** eine C₁₋₃-Alkylgruppe und **R⁶** eine C₁₋₆-Alkylgruppe bedeuten, wird zweckmäßigerweise in einem Lösungsmittel wie Toluol, Chlorbenzol, Dimethylformamid, Dimethylsulfoxid (DMSO), Dichlormethan, Acetonitril oder Pyridin, beispielsweise bei Temperaturen zwischen 0°C und 150°C und zweckmäßigerweise in Gegenwart von Basen wie Pyridin, Triethylamin, DIPEA, Kaliumcarbonat, Kalium-tert-butylat oder Natriummethanolat durchgeführt, wobei das Alkylsulfonat als Abgangsgruppe dient.

Die Hydrolyse der Carbonsäureester der allgemeinen Formel **XIX** zu Carbonsäuren der allgemeinen Formel **XX** erfolgt wie unter Schema 2 beschrieben.

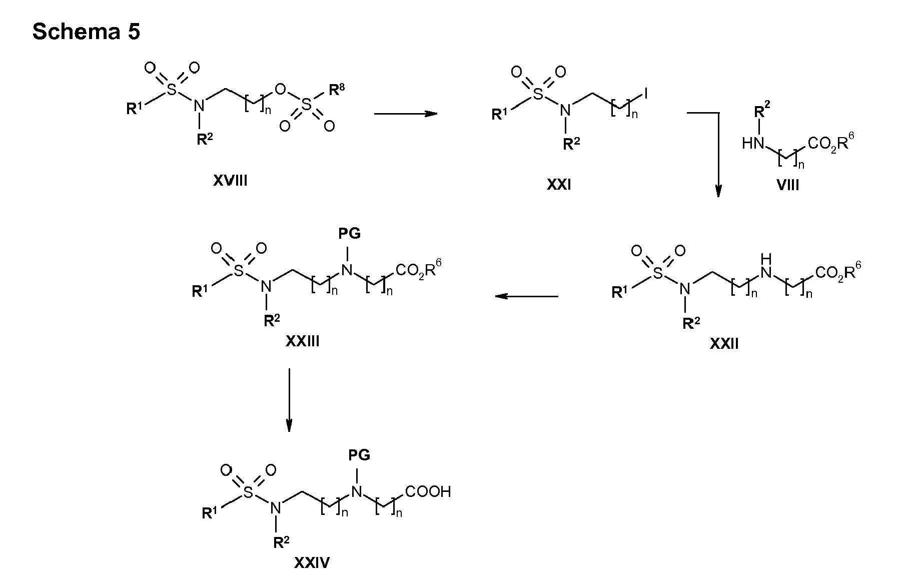

Die Finkelsteinstein-Reaktion von Verbindungen der allgemeinen Formel **XVIII,** in der **R¹** und **R²** wie eingangs erwähnt definiert sind, n eine Zahl 1, 2, 3 oder 4 und **R⁶** eine C₁₋₃-Alkylgruppe und **R⁸** eine C₁₋₃-Alkylgruppe oder eine ggf. durch C₁₋₃-Alkylgruppe substituierte Phenylgruppe bedeuten, zu Halogeniden der allgemeinen Formel XXI, in der **R¹** und **R²** wie eingangs erwähnt definiert sind und n eine Zahl 1, 2, 3 oder 4 und **R⁶** eine C₁₋₃-Alkylgruppe bedeutet, erfolgt unter bekannten Reaktionsbedingungen (siehe z.B. H. Finkelstein, Berichte der Deutschen Chemischen Gesellschaft 43, 1910, 1528).

Die anschließende Alkylierung des Glycinesters wird wie unter Schema 4 beschrieben durchgeführt (**R²** ≠ H).

Die Aminofunktion in den Verbindungen der allgemeinen Formel **XXIII** wird durch eine konventionelle Schutzgruppe **PG** nach bekannten Verfahren geschützt. Die ausgewählte Schutzgruppe ist eine, die unter nicht-hydrogenolytischen Bedingungen abgespalten werden kann. Eine bevorzugte Schutzgruppe ist die Boc-Gruppe. Eine Übersicht über die Chemie der Schutzgruppen findet sich in Theodora W. Greene und Peter G. M. Wuts, Protective Groups in Organic Synthesis, Second Edition, 1991, Verlag John Wiley and Sons sowie in Philip J. Kocieński, Protecting Groups, 3rd Edition, 2005, Georg Thieme Verlag.

Die Spaltung der Carbonsäureester der allgemeinen Formel **XXIII** zu Carbonsäuren der allgemeinen Formel **XXIV** erfolgt wie unter Schema 2 beschrieben.

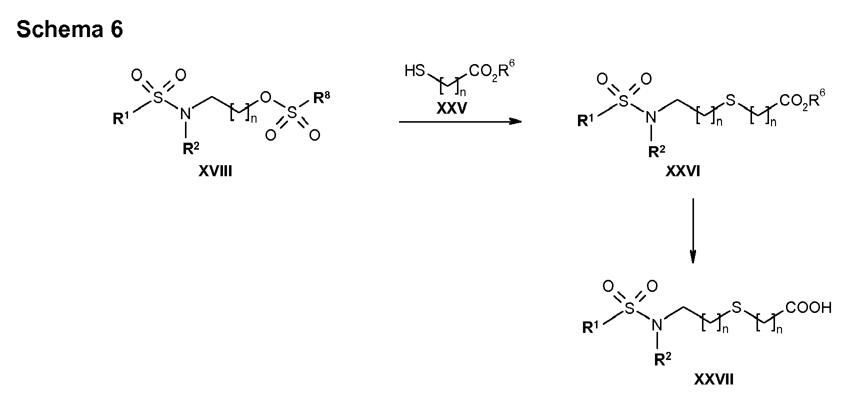

Die Alkylierung eines Thiols der allgemeinen Formel **XXV**, in der n eine Zahl 1, 2, 3 oder 4 und **R⁶** eine C₁₋₆-Alkylgruppe bedeutet, zu Verbindungen der allgemeinen Formel **XXVI**, in der **R¹** und **R²** wie voranstehend erwähnt definiert sind, n eine Zahl 1, 2, 3 oder 4 und **R⁶** eine C₁₋₆-Alkylgruppe bedeuten, wird zweckmäßigerweise in einem Lösungsmittel wie Toluol, Chlorbenzol, DMF, DMSO, Dichlormethan, Acetonitril oder Pyridin, beispielsweise bei Temperaturen zwischen 0°C und 150°C und zweckmäßigerweise in Gegenwart von Basen wie Pyridin, Triethylamin, DIPEA, Kaliumcarbonat, Kalium-tert-butylat oder Natriummethanolat durchgeführt, wobei das Alkylsulfonat als Abgangsgruppe dient.

Die Hydrolyse der Carbonsäureester der allgemeinen Formel **XXVI** zu Carbonsäuren der allgemeinen Formel **XXVII,** in der alle Reste wie voranstehend erwähnt definiert sind, erfolgt wie unter Schema 2 beschrieben.

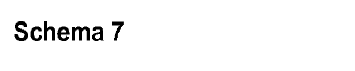

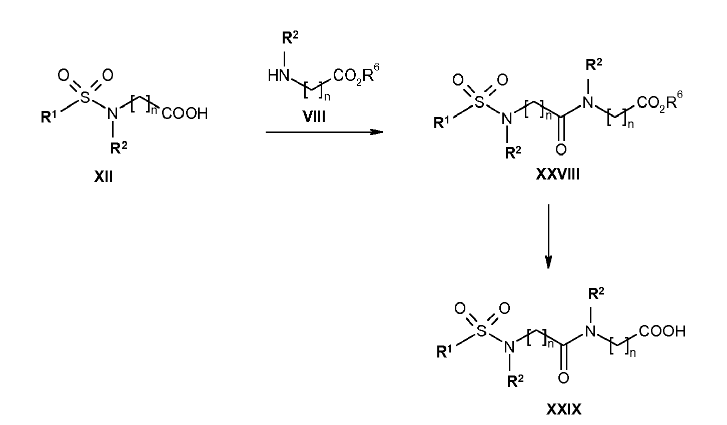

Die Amidknüpfung von Carbonsäuren der allgemeinen Formel **XII,** in der **R¹** und **R²** wie voranstehend erwähnt definiert sind und n eine Zahl 1, 2, 3 oder 4 bedeutet, und Aminosäuren der allgemeinen Formel **VIII,** in der **R¹** und **R²** wie voranstehend erwähnt definiert sind, **n** eine Zahl 1, 2, 3 oder 4 und **R⁶** eine C₁₋₆-Alkylgruppe bedeuten, zu Carbonsäureamiden der allgemeinen Formel **XXVIII,** in der **R¹** und **R²** wie voranstehend erwähnt definiert sind, **n** eine Zahl 1, 2, 3 oder 4 und **R⁶** eine C₁₋₆-Alkylgruppe bedeuten, wird wie unter Schema 1 beschrieben durchgeführt.

Wie unter Schema 2 ausgeführt, wird der Carbonsäureester der allgemeinen Formel **XXVIII** zu Carbonsäure der allgemeinen Formel **XXIX**, in der **R¹** und **R²** wie voranstehend erwähnt definiert sind und n eine Zahl 1, 2, 3 oder 4 bedeutet, gespalten.

Die als Ausgangsstoffe verwendeten Amine der allgemeinen Formel **IV** sind entweder käuflich erwerbbar, oder man erhält sie nach an sich literaturbekannten Verfahren, beispielsweise durch die in Schema 8 bis 12 dargestellten Synthesewege, wobei **R^{1.1}** wie voranstehend erwähnt definiert ist, **Hal¹** ein Chlor- oder Bromatom und **Hal²** ein Fluor-, Chlor- oder Bromatom oder einen Rest **R⁹** bedeuten.

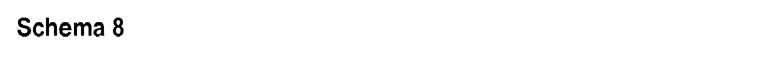

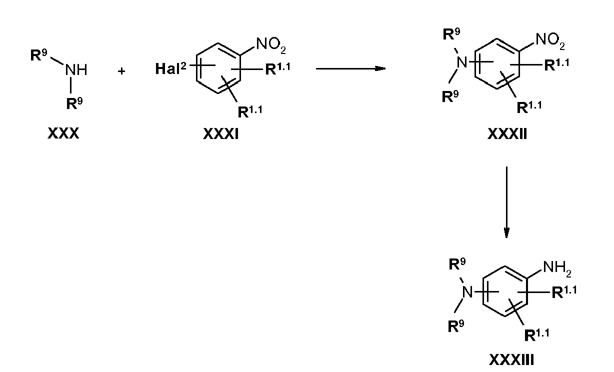

Die Reaktion eines Amins der allgemeinen Formel **XXX**, in der **R⁹** eine C₁₋₃-Alkylgruppe bedeutet, mit einem Halogen-nitrobenzol der allgemeinen Formel **XXXI**, in der **R^{1.1}** wie voranstehend erwähnt definiert ist und **Hal²** ein Fluor-, Chlor- oder Bromatom oder einen Rest **R⁹** bedeutet, erfolgt nach bekannten Verfahren, beispielsweise in einem Lösungsmittel wie Tetrahydrofuran, Dimethylformamid oder Dimethylsulfoxid und zweckmäßigerweise in Gegenwart einer passenden Base wie Triethylamin oder Kaliumcarbonat, bei einer Temperatur von 20°C bis 160°C. Ist das Amin der allgemeinen Formel **XXX** flüssig, kann die Reaktion auch ohne Lösungsmittel und zusätzlicher Base durchgeführt werden.

Die Reduktion der Nitrogruppe zu Anilinen der allgemeinen Formel **XXXIII,** in der **R^{1.1}** wie voranstehend erwähnt definiert ist und **R⁹** eine C₁₋₃-Alkylgruppe bedeutet, erfolgt nach Standard-Reaktionsbedingungen (siehe z.B. Richard C. Larock, Comprehensive Organic Transformations, 1989, VCH), vorzugsweise nach Standard-Bedingungen der katalytischen Hydrogenolyse mit einem Katalysator wie Palladium auf Kohle oder Raney-Nickel in einem Lösungsmittel wie Methanol oder Ethanol.

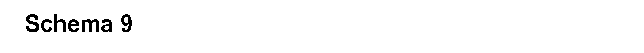

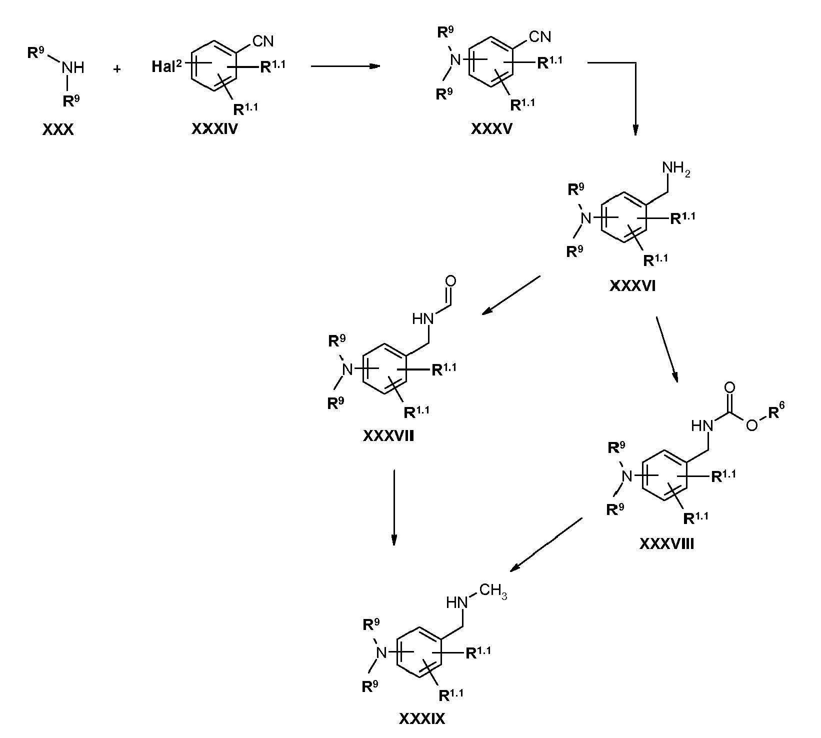

Die Umsetzung von Verbindungen der allgemeinen Formeln **XXX**, in der **R⁹** eine

C₁₋₃-Alkylgruppe bedeutet, mit einer Verbindung der allgemeinen Formel **XXXIV**, in der **R^{1.1}** wie voranstehend erwähnt definiert ist und **Hal²** ein Fluor-, Chlor- oder Bromatom oder einen Rest **R⁹** bedeutet, zu Verbindungen mit der allgemeinen Formel **XXXV**, in der **R^{1.1}** wie voranstehend erwähnt definiert ist und **R⁹** eine C₁₋₃-Alkylgruppe bedeutet, wird wie unter Schema 8 beschrieben durchgeführt.

Die Reduktion eines Nitrils der allgemeinen Formel **XXXV** zu einem Amin der allgemeinen Formel **XXXVI,** in der **R^{1.1}** wie voranstehend erwähnt definiert ist und **R⁹** eine C₁₋₃-Alkylgruppe bedeutet, kann unter Standard-Bedingungen der katalytischen Hydrogenolyse mit einem Katalysator wie beispielsweise Raney-Nickel in einem Lösungsmittel wie ammoniakalischem Methanol oder Ethanol oder mit einem Reduktionsmittel wie Lithiumaluminiumhydrid oder Natriumborhydrid in einem Lösungmittel wie Tetrahydrofuran, gegebenenfalls in Gegenwart von Aluminiumchlorid, durchgeführt werden.

Die Formylierung eines Amins der allgemeinen Formel **XXXVI** zu einer Verbindung der allgemeinen Formel **XXXVII**, in der **R^{1.1}** wie voranstehend erwähnt definiert ist und **R⁹** eine C₁₋₃-Alkylgruppe bedeutet, erfolgt zweckmäßigerweise in einem Lösungsmittel wie Dichlormethan, beispielsweise bei Temperaturen von 40°C bis 70°C und in Gegenwart von Essigsäureanhydrid und Ameisensäure.

Die Carbamatbildung zu Verbindungen der allgemeinen Formel **XXXVIII,** in der **R^{1.1}** wie voranstehend erwähnt definiert ist, **R⁶** eine C₁₋₆-Alkyl- und **R⁹** eine C₁₋₃-Alkylgruppe bedeutet, wird nach bekannten Verfahren durchgeführt, beispielsweise mit einem Chlorameisensäureester oder Boc-Anhydrid in Gegenwart einer Base wie Triethylamin oder Natronlauge und einem Lösungsmittel wie THF oder Dioxan.

Die Reduktion des Formyls bzw. des Carbamates zu Verbindungen der allgemeinen Formel **XXXIX,** in der **R^{1.1}** wie voranstehend erwähnt definiert ist und **R⁹** eine C₁₋₃-Alkylgruppe bedeutet, erfolgt unter Standard-Reaktionsbedingungen, vorzugsweise mit einem Reduktionsmittel wie Lithiumaluminiumhydrid und in einem Lösungsmittel wie Tetrahydrofuran bei einer Temperatur von 50°C bis 100°C.

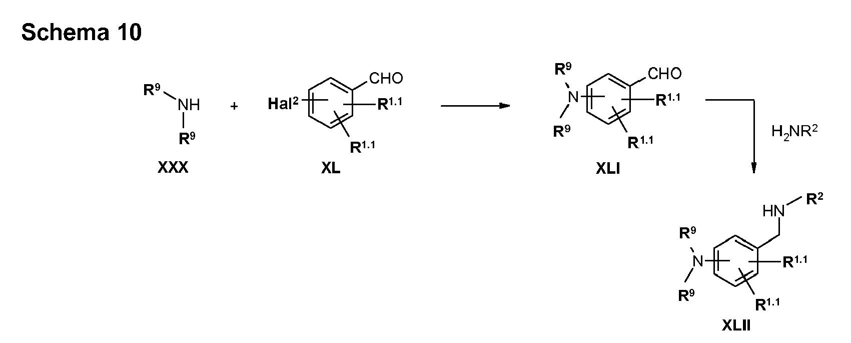

Der Halogen-Stickstoff-Austausch in Verbindungen der allgemeinen Formeln **XXX**, in der **R⁹** eine C₁₋₃-Alkylgruppe bedeutet, und **XL**, in der **R^{1.1}** wie voranstehend erwähnt definiert ist und **Hal²** ein Fluor-, Chlor- oder Bromatom oder einen Rest **R⁹** bedeutet, zur Herstellung von Verbindungen der allgemeinen Formel **XLI**, in der **R^{1.1}** wie voranstehend erwähnt definiert ist und **R⁹** eine C₁₋₃-Alkylgruppe bedeutet, wird wie unter Schema 8 beschrieben durchgeführt.

Die Reaktion von Benzaldehyden der allgemeinen Formel **XLI**, in der **R^{1.1}** wie voranstehend erwähnt definiert ist und **R⁹** eine C₁₋₃-Alkylgruppe bedeutet, mit einem Amin der allgemeinen Formel H₂N**R²**, in der **R²** wie voanstehend erwähnt definiert ist, zu einer Verbindung der allgemeinen Formel **XLII,** in der **R^{1.1}** und **R²** wie voranstehend erwähnt definiert sind und **R⁹** eine C₁₋₃-Alkylgruppe bedeutet, ist eine reduktive Aminierung. Sie erfolgt nach bekannten Verfahren, beispielsweise mit einem Reduktionsmittel wie Natriumtriacetoxyborhydrid, Natriumborhydrid oder Natriumcyanoborhydrid, zweckmäßigerweise in einem Lösungsmittel wie Tetrahydrofuran oder Dichlormethan, gegebenenfalls unter Zusatz von Essigsäure.

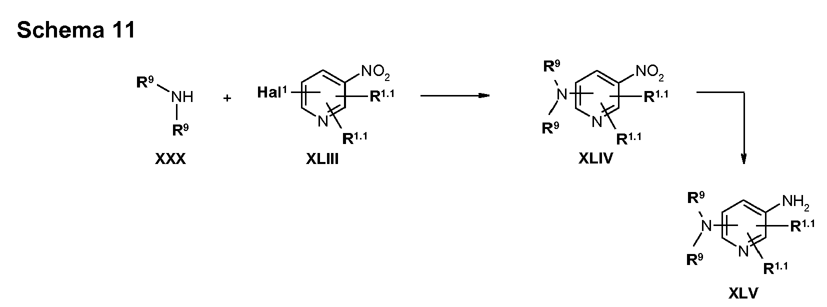

Die Reaktion eines Amins der allgemeinen Formel **XXX**, in der **R⁹** eine C₁₋₃-Alkylgruppe bedeutet, mit einem Halogen-nitropyridin der allgemeinen Formel **XLIII,** in der **R^{1.1}** wie voranstehend erwähnt definiert ist und **Hal¹** ein Chlor- oder Bromatom bedeutet, erfolgt nach bekannten Verfahren, beispielsweise in einem Lösungsmittel wie Tetrahydrofuran, Dichlormethan, Methanol oder DMSO und zweckmäßigerweise in Gegenwart einer passenden Base wie Triethylamin, Natronlauge oder Kaliumcarbonat und bei einer Temperatur von 20°C bis 100°C.

Die anschließende Reduktion der Nitrogruppe einer Verbindung der allgemeinen Formel **XLIV,** in der **R^{1.1}** wie voranstehend erwähnt definiert ist und **R⁹** eine C₁₋₃-Alkylgruppe bedeutet, zu Verbindungen der allgemeinen Formel **XLV**, in der **R^{1.1}** wie voranstehend erwähnt definiert ist und **R⁹** eine C₁₋₃-Alkylgruppe bedeutet, wird wie unter Schema 8 beschrieben durchgeführt.

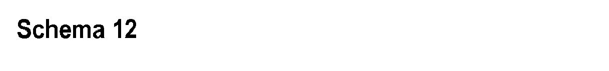

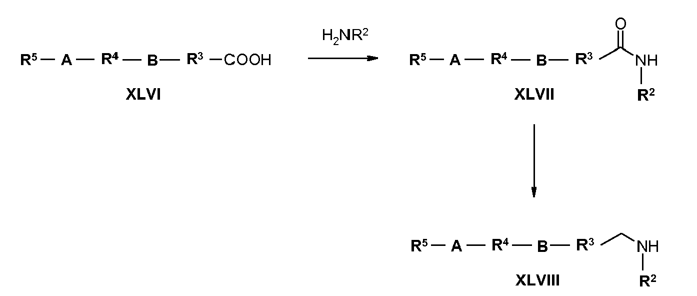

Die Amidknüpfung von Carbonsäuren der allgemeinen Formel **XLVI**, in der alle Reste wie voranstehend erwähnt definiert sind, und Aminen der allgemeinen Formel H₂NR², in der **R²** wie voranstehend erwähnt definiert ist, zu Carbonsäureamiden der allgemeinen Formel **XLVII**, in der alle Reste wie voranstehend erwähnt definiert sind, wird wie unter Schema 1 beschrieben durchgeführt.

Die Reduktion von Carbonsäureamiden der allgemeinen Formel **XLVII** zu Aminen der allgemeinen Formel **XLVIII,** in der alle Reste wie voranstehend erwähnt definiert sind, erfolgt nach Standard-Reaktionsbedingungen, vorzugsweise in Gegenwart eines Reduktionsmittels wie Lithiumaluminiumhydrid und einem Lösungsmittel wie Tetrahydrofuran bei 40°C bis 100°C.

### Methodenbeschreibung zur hBK1-Rezeptorbindung

CHO-Zellen, die den hBK1-Rezeptor exprimieren, werden in "Dulbecco's modified Medium" kultiviert. Von konfluenten Kulturen wird das Medium entfernt, die Zellen werden mit PBS-Puffer gewaschen, abgeschabt und durch Zentrifugieren isoliert. Anschließend werden die Zellen in Suspension homogenisiert, das Homogenat zentrifugiert und resuspendiert. Nach Bestimmung des Proteingehalts wird die so erhaltene Membranpräparation bei -80°C eingefroren.

Nach dem Auftauen werden 200 µl des Homogenats (50 bis100 µg Protein/Assay) für 60 Minuten bei Raumtemperatur mit 0.5 bis 1.0 nM Kallidin (DesArg10,Leu9), [3,4-Prolyl-3,43H(N)] und ansteigenden Konzentrationen der Testsubstanz in einem Gesamtvolumen von 250 µl inkubiert. Die Inkubation wird durch rasche Filtration durch GF/B-Glasfaserfilter, die mit Polyethyleneimine (0.3%) vorbehandelt wurden, beendet. Die an das Protein gebundene Radioaktivität wird mit einem TopCount NXT gemessen. Als nichtspezifische Bindung wird die gebundene Radioaktivität in Gegenwart von 1.0 µM Kallidin (DesArg10,Leu9), [3,4-Prolyl-3,43H(N)] definiert. Die Analyse der Konzentrations-Bindungskurve erfolgt mit Hilfe einer computergestützten nichtlinearen Kurvenanpassung. Aus den so erhaltenen Daten wird für die Testsubstanz der entsprechende Kᵢ - Wert ermittelt.

Um zu zeigen, dass die Verbindungen der allgemeinen Formel **I** mit unterschiedlichen Strukturelementen gute bis sehr gute Bradikinin-B1-Rezeptor antagonistische Aktivitäten zeigen, werden in der folgenden Tabelle die Kᵢ-Werte angegeben, die gemäß dem voranstehenden Versuchsprotokoll erhalten wurden. Dazu wird angemerkt, dass die Verbindungen im Hinblick auf ihre unterschiedlichen strukturellen Elemente ausgewählt wurden und nicht um spezifische Verbindungen hervorzuheben:

| | |
|---|---|
| **Beispiel** | **Kⱼ [nM]** |
| (1) | 8.7 |
| (3) | 5.1 |

### INDIKATIONEN

Aufgrund ihrer pharmakologischen Eigenschaften eignen sich die neuen Verbindungen und deren physiologisch verträglichen Salze zur Behandlung von Krankheiten und Krankheitssymptomen, die wenigstens teilweise durch die Stimulierung von Bradykinin-B1-Rezeptoren hervorgerufen werden.

Aufgrund ihrer pharmakologischen Wirkung eignen sich die Substanzen zur Behandlung
(a) von **akuten Schmerzen** wie z.B. Zahnschmerzen, peri- und postoperativen Schmerzen, traumatischen Schmerzen, Muskelschmerzen, Verbrennungsschmerzen, Schmerzen bei Sonnenbrand, Trigeminusneuralgie, Schmerzen bei Koliken, sowie bei Spasmen des Magen-Darm-Trakts oder des Uterus;
(b) von **Eingeweideschmerzen** wie z.B. chronischen Beckenschmerzen, gynäkologischen Schmerzen, Schmerzen vor und während der Menstruation, Schmerzen bei Pankreatitis, bei peptischen Ulzera, bei interstitieller Zystitis, bei Nierenkolik, bei Angina pectoris, Schmerzen bei Kolon irritabile, bei nicht-ulzeröser Dyspepsie und bei Gastritis, nicht-kardialen Thoraxschmerzen und Schmerzen bei myokardialer Ischämie und Herzinfarkt;
(c) von **neuropathischen Schmerzen** wie z.B. schmerzhaften Polyneuropathien, Schmerzen bei diabetischer Neuropathie, AIDS-assoziierten neuropathischen Schmerzen, Schmerzen bei Lumbago, bei nicht-herpesassoziierter Neuralgie, bei Post-zoster Neuralgie, bei Nervenverletzungen, bei Schädel-Hirn-Trauma, Schmerzen bei Nervenchädigungen infolge von Toxinen oder Chemotherapie, Phantomschmerzen, Schmerzen bei multipler Sklerose, bei Nervenwurzelabriß und schmerzhaften traumatisch bedingten Einzelnervenschädigungen;
(d) von **entzündlichen / Schmerzrezeptor-vermittelten Schmerzen** im Zusammenhang mit Erkrankungen wie Osteoarthritis, rheumatoider Arthritis, rheumatischem Fieber, Tendo-Synovitis, Sehnenentzündungen, Gicht, Vulvodynie, Schädigungen und Erkrankungen der Muskeln und Faszien (muskuläre Verletzungen, Fibromyalgie), Osteoarthritis, juveniler Arthritis, Spondylitis, Gicht-Arthritis, Psoriasis-Arthritis, Fibromyalgie, Myositis, Migräne, Zahnerkrankungen, Influenza und anderen Virusinfektionen wie Erkältungen, systemischer Lupus erythematodes,
(e) von **Tumorschmerzen** im Zusammenhang mit Krebserkrankungen wie lymphatischer oder myeloischer Leukämie, Hodgkin Erkrankung, Non-Hodgkin Lymphomen, Lymphogranulomatose, Lymphosarkomen, soliden malignen Tumoren und ausgedehnten Metastasen;
(f) von **Kopfschmerz-Erkrankungen** wie z.B. Kopfschmerzen unterschiedlicher Ursache, Cluster-Kopfschmerz, Migräne (mit oder ohne Aura) und Spannungskopfschmerz.
   Weiterhin eigenen sich die Verbindungen zur Behandlung
(g) von entzündlichen Veränderungen im Zusammenhang mit Erkrankungen der Atemwege wie Asthma bronchiale, einschließlich allergischem Asthma (atopisch und nicht-atopisch) sowie Bronchospasmen bei Anstrengung, beruflich-bedingtem Asthma, viraler oder bakterieller Exazerbation einer bestehenden Asthma-Erkrankung und anderen nicht-allergisch bedingten asthmatischen Erkrankungen; chronisch obstruktiver Lungen-Erkrankung (COPD) einschließlich Lungenemphysem, akutem Atemnotsyndrom des Erwachsenen (ARDS), Bronchitis, Lungenentzündung, allergischer Rhinitis (saisonal und ganzjährig), vasomotorischer Rhinitis und Staublungen-Erkrankungen wie Aluminose, Anthrakose, Asbestose, Chalikose, Siderose, Silikose, Tabakose und Byssinose;
(h) von Entzündungserscheinungen bei Sonnenbrand und Verbrennungen, Ödemen nach Traumata durch Verbrennungen, Hirnödemen und Angioödemen, Darmerkrankungen einschließlich Morbus Crohn und Kolitis ulzerosa, Reizdarmsyndrom, Pankreatitis, Nephritis, Zystitis (interstitielle Zystitis), Uveitis; entzündlichen Erkrankungen der Haut (wie z.B. Psoriasis und Ekzeme), vaskulären Bindegewebserkrankungen, Lupus, Zerrungen und Frakturen;
(i) von Diabetes Mellitus und dessen Folgen (wie z.B. diabetische Vaskulopathie, diabetische Neuropathie, diabetische Retinopathie) und von diabetischen Symptomen bei Insulitis (z.B. Hyperglycämie, Diurese, Proteinurie und erhöhte renale Nitrit- und Kallikrein-Exkretion);
(j) von neurodegenerativen Erkrankungen wie der Parkinson schen Erkrankung und der Alzheimer Erkrankung;
(k) von Sepsis und septischem Schock nach bakteriellen Infektionen oder nach Trauma;
(l) von Juckreiz verursachenden Syndromen und allergischen Hautreaktionen;
(m) von Osteoporose;
(n) von Epilepsie;
(o) von Verletzungen des Zentralnervensystems;
(p) von Wunden und Gewebeschädigungen;
(q) von Zahnfleischentzündungen;
(r) von benigner Prostata-Hyperplasie und hyperaktiver Blase;
(s) von Pruritus;
(t) von Vitiligo;
(u) von Störungen der Motilität von respiratorischen, genito-urinalen, gastro-intestinalen oder vaskulären Regionen und
(v) von post-operativem Fieber.

Neben der Eignung als Humantherapeutika, sind diese Substanzen auch nützlich in der veterinärmedizinischen Therapie von Haustieren, exotischen Tieren und Nutztieren.

### KOMBINATIONEN

Zur Behandlung von Schmerzen kann es vorteilhaft sein, die erfindungsgemässen Verbindungen mit belebenden Stoffen wie Koffein oder anderen schmerzlindernden Wirkstoffen zu kombinieren. Stehen zur Behandlung der Ursache der Schmerzen geeignete Wirk- stoffe zur Verfügung, so können diese mit den erfindungsgemässen Verbindungen kombiniert werden. Sind unabhängig von der Schmerzbehandlung auch noch weitere medizinische Behandlungen angezeigt, zum Beispiel gegen Bluthochdruck oder Diabetes, so können auch die dafür nötigen Wirkstoffe mit den erfindungsgemässen Verbindungen kombiniert werden.

Für eine Kombinationstherapie kommen beispielsweise die folgenden Verbindungen in Frage:
Nicht-steroidale Antirheumatika (NSAR): COX-2 Hemmer wie Propionsäure-Derivate (Alminoprofen, Benoxaprofen, Bucloxinsäure, Carprofen, Fenhufen, Fenoprofen, Fiuprofen, Fiulbiprofen, Ibuprofen, Indoprofen, Ketoprofen, Miroprofen, Naproxen, Oxaprozin, Pirprofen, Pranoprofen, Suprofen, Tiaprofensäure, Tioxaprofen), Essigsäure-Derivate (Indomethacin, Acemetacin, Alcofenac, Isoxepac, Oxpinax, Sulindac, Tiopinac, Tolmetin, Zidometacin, Zomepirac) Fenaminsäure-Derivate (Meclofenaminsäure, Mefenaminsäure, Tolfenaminsäure), Biphenyl-Carboxylsäure-Derivate, Oxicame (Isoxicam, Meloxicam, Piroxicam, Sudoxicam und Tenoxicam), Salicylsäure-Derivate (Acetylsalicylsäure, Sulfasalazin, warum nicht auch Mesalazin, Olsalazin, und Pyrazolone (Apazon, Bezpiperylon, Feprazon, Mofebutazon, Oxyphenbutazon, Phenylbutazon, warum nicht auch Propyphenazon und Metamizol, und Coxibe (Celecoxib, Valecoxib, Rofecoxib, Etoricoxib).
   Opiat Rezeptor Agonisten wie z. B. Morphin, Propoxyphen (Darvon), Tramadol, Buprenorphin.
   Cannabinoid Agonisten wie z.B. GW-1000, KDS-2000, SAB-378, SP-104, NVP001-GW-843166, GW-842166X, PRS-211375.
   Natriumkanalblocker wie z.B. Carbamazepin, Mexiletin, Lamotrigin, Pregabalin, Tectin,
   NW-1029, CGX-1002.
   N-Typ Calciumkanalblocker wie z.B. Ziconitid, NMED-160, SP1-860.
   Serotonerge und noradrenerge Modulatoren wie z.B. SR-57746, Paroxetin, Duloxetin, Clonidin, Amitriptylin, Citalopram.
   Corticosteroide wie z.B. Betamethason, Budesonid, Cortison, Dexamethason, Hydrocortison, Methylprednisolon, Prednisolon, Prednison und Triamcinolon.
   Histamin H1-Rezeptorantagonisten wie z.B. Bromophtniramint, Chlorpheniramin, Dexchlorpheniramin, Triprolidin, Clemastin, Diphenhydramin, Diphenylpyralin, Tripelennamin, Hydroxyzin, Methdijazin, Promethazin, Trimeprazin Azatadin, Cyproheptadin, Antazolin, Pheniramin, Pyrilamin, Astemizol, Terfenadin, Loratadin,
   Cetirizin, Desloratadin, fexofenadin, Levocetirizin.
   Histamin H2-Rezeptor Antagonisten wie z.B. Cimetidin, Famotidin, und Ranitidin. Protonenpumpenhemmer wie z.B. Omeprazol, Pantoprazol, Esomeprazol.
   Leukotrien Antagonisten und 5-Lipoxygenasehemmer wie z.B. Zafirlukast, Montelukast, Pranlukast und Zileuton.
   Lokalanästhetika wie z.B. Ambroxol, Lidocain.
   VR1 Agonisten und Antagonisten wie z.B. NGX-4010, WL-1002, ALGRX-4975, WL-10001, AMG-517.
   Nikotinrezeptor Agonisten wie z.B. ABT-202, A-366833, ABT-594, BTG-102, A-85380,
   CGX1204.
   P2X3-Rezeptor Antagonisten wie z.B. A-317491, ISIS-13920, AZD-9056.
   NGF Agonisten und Antagonisten wie z.B. RI-724, RI-1024, AMG-819, AMG-403, PPH 207.
   NK1 und NK2 Antagonisten wie z.B. DA-5018, R-116301, CP-728663, ZD-2249.
   NMDA Antagonisten wie z.B. NER-MD-11, CNS-5161, EAA-090, AZ-756, CNP-3381. Kaliumkanal Modulatoren wie z.B. CL-888, ICA-69673, Retigabin.
   GABA Modulatoren wie z.B. Lacosamid.
   Serotonerge und noradrenerge Modulatoren wie z.B. SR-57746, Paroxetin, Duloxetin, Clonidin, Amitriptylin, Citalopram, Flibanserin.
   Anti-Migräne Therapeutika wie z.B. Sumatriptan, Zolmitriptan, Naratriptan, Eletriptan.
Die zur Erzielung einer schmerzstillenden Wirkung erforderliche Dosierung beträgt bei intravenöser Gabe zweckmäßigerweise 0.01 bis 3 mg/kg Körpergewicht, vorzugsweise 0.1 bis 1 mg/kg, und bei oraler Gabe 0.1 bis 8 mg/kg Körpergewicht, vorzugsweise 0.5 bis 3 mg/kg, jeweils 1 bis 3 x täglich. Die erfindungsgemäß hergestellten Verbindungen können intravenös, subkutan, intramuskulär, intrarektal, intranasal, durch Inhalation, transdermal oder oral verabreicht werden, wobei zur Inhalation insbesondere Aerosolformulierungen geeignet sind. Sie können gegebenenfalls zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragees, Kapseln, Pulver, Suspensionen, Lösungen, Dosieraerosole oder Zäpfchen eingearbeitet werden.

### EXPERIMENTELLER TEIL

Für die hergestellten Verbindungen liegen in der Regel IR-, ¹H-NMR und/oder Massenspektren vor. Die bei den Fliessmitteln angegebenen Verhältnisse beziehen sich auf Volumeneinheiten der jeweiligen Lösungsmittel. Die angegebenen Volumeneinheiten bei Ammoniak beziehen sich auf eine konzentrierte Lösung von Ammoniak in Wasser.
Soweit nicht anders vermerkt sind die bei den Aufarbeitungen der Reaktionslösungen verwendeten Säure-, Basen- und Salzlösungen wässrige Systeme der angegebenen Konzentrationen.
Zu chromatographischen Reinigungen wird Kieselgel der Firma Millipore (MATREX*^{™}*, 35-70 µm) oder Alox (E. Merck, Darmstadt, Aluminiumoxid 90 standardisiert, 63-200 µm, Artikel-Nr: 1.01097.9050) verwendet.

### In den Versuchsbeschreibungen werden die folgenden Abkürzungen verwendet:

| | |
|---|---|
| CDI | 1,1'-Carbonyldiimidazol |
| DC | Dünnschichtchromatogramm |
| DIPEA | Diisopropylethylamin |
| DMAP | 4-Dimethylaminopyridin |
| DMF | Dimethylformamid |
| DMSO | Dimethylsulfoxid |
| HATU | *O*-(7-Azabenzotriazol-1-yl)-*N*,*N*,*N*',*N*'-tetramethyluroniumhexafluorphosphat |
| tert | tertiär |
| TBTU | 2-(1 *H*-Benzotriazol-1-yl)-1,1,3,3-tetramethyluronium-tetrafluorborat |
| THF | Tetrahydrofuran |

### Folgende analytische HPLC-Methode wurde verwendet:

| | | |
|---|---|---|
| Methode 1: | Säule: | Zorbax Stable Bond C18, 3.5 µM, 4.6 x 75 mm |
| | Detektion: | 230 - 360 nm |
| | Fließmittel A: | Wasser / 0.1 % Ameisensäure |

### Fließmittel B: Acetonitril / 0.1 % Ameisensäure

### Gradient:

| Zeit in min | %A | %B | Flussrate in mL/min |
|---|---|---|---|
| 0.0 | 95.0 | 5.0 | 1.6 |
| 0.1 | 95.0 | 5.0 | 1.6 |
| 4.5 | 10.0 | 90.0 | 1.6 |
| 5.09 | 10.0 | 90.0 | 1.6 |
| 5.5 | 90.0 | 10.0 | 1.6 |

### Methode 2: Säule: Merck Cromolith Speed ROD RP18e, 4.6 x 50 mm

### Detektion: 190 - 400nm

### Fließmittel A: Wasser / 0.1 % Ameisensäure

### Fließmittel B: Acetonitril / 0.1 % Ameisensäure

### Gradient:

| Zeit in min | %A | %B | Flussrate in mL/min |
|---|---|---|---|
| 0.0 | 90.0 | 10.0 | 1.5 |
| 4.5 | 10.0 | 90.0 | 1.5 |
| 5.0 | 10.0 | 90.0 | 1.5 |
| 5.5 | 90.0 | 10.0 | 1.5 |

### Die folgende HPLC-Methode wurde zur präparativen Enantiomerentrennung verwendet:

| | | |
|---|---|---|
| Methode 3: | Säule: | Daicel OJ-H, 250 x 4.6 mm, 5 µ |
| | Detektion: | 230 - 360 nm |
| | Fließmittel: | n-Hexan + 0.2 % Diethylamin / Ethanol = 70:30 |
| | Flußrate: | 12 ml/min |
| | Gradient: | isokratisch |

### Folgende Mikrowellen-Apparatur wurde verwendet: Biotage EmrysOptimizer^{™}

### Herstellung der Endverbindungen

### Beispiel 1

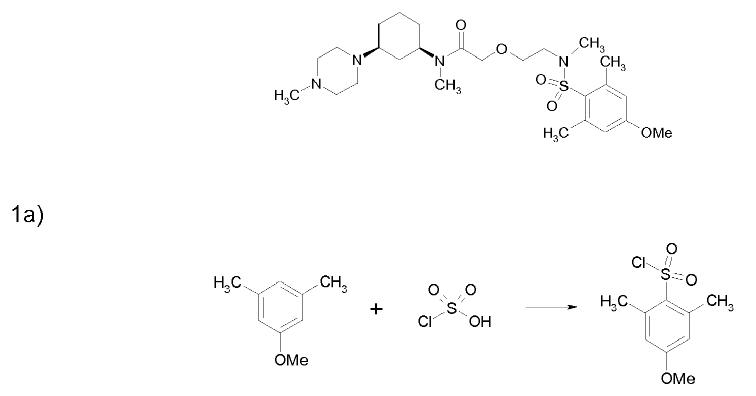

Eine Mischung aus 2.0 g (14.69 mmol) 3,5-Dimethylanisol und 20 ml Dichlormethan wird unter Eisbadkühlung mit 5.85 ml (88.0 mmol) Chlorsulfonsäure versetzt. Die Reaktionsmischung wird danach 20 min bei Raumtemperatur gerührt und anschließend auf 50 ml Eiswasser gegossen. Man extrahiert mit 100 ml Dichlormethan. Die organischen Extrakte werden mit 5%iger Natriumhydrogencarbonat-Lösung gewaschen, über Natriumsulfat getrocknet und bis zur Trockene eingeengt.
C₉H₁₁ClO₃S (234.70)
[M+H]+=234/236
DC: Kieselgel, Petrolether / Ethylacetat 9:1, Rf-Wert = 0.46

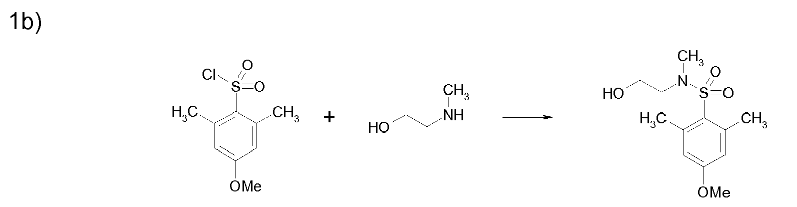

1.69 g (21.1 mmol) N-Methylaminoethanol (BASF) und 6.68 ml (47.9 mmol) Triethylamin werden in 100 ml Dichlormethan gelöst. Bei 0°C werden 4.50 g (19.2 mmol) Produkt aus 1a gelöst in 50 ml Dichlormethan zugetropft. Man entfernt die Kühlung und rührt 1.5 Stunden bei Raumtemperatur. Das Reaktionsgemisch wird anschließend mit 1 N Salzsäure und 5-prozentiger Natriumhydrogencarbonatlösung gewaschen. Die organische Phase wird über Natriumsulfat getrocknet und bis zur Trockene eingeengt.
C₁₂H₁₉NO₄S (273.35)
[M+H]+=274
DC: Kieselgel, Dichlormethan / Ethanol 19:1, Rf-Wert = 0.43

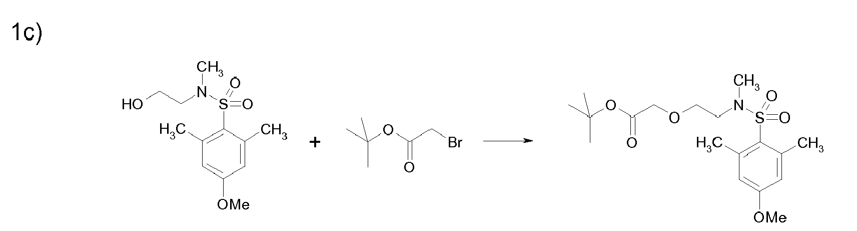

Eine Mischung aus 5.15 g (18.8 mmol) Produkt aus 1 b, 1.75 g (6.60 mmol) Tetrabutylammoniumchlorid (Fluka) und 80 ml Toluol wird bei 0°C zuerst mit 100 ml 35-prozentiger Natronlauge, dann mit 4.18 ml (28.3 mmol) Bromessigsäure-tert-butylester in 20 ml Toluol versetzt. Die Reaktionsmischung wird anschließend 1.5 Stunden bei Raumtemperatur gerührt, dann mit Diethylether verdünnt. Nach der Phasentrennung wird die organische Phase viermal mit Wasser neutral gewaschen, über Natriumsulfat getrocknet und im Vakuum bis zur Trockene eingeengt. Das so erhaltene Rohprodukt wird durch Säulenchromatographie über Kieselgel (Elutionsmittel: Petrolether / Ethylacetat 4:1) gereinigt.
C₁₈H₂₉NO₆S (387.49)
[M+H]+=388
DC: Kieselgel, Petrolether / Ethylacetat 7:3, Rf-Wert = 0.59

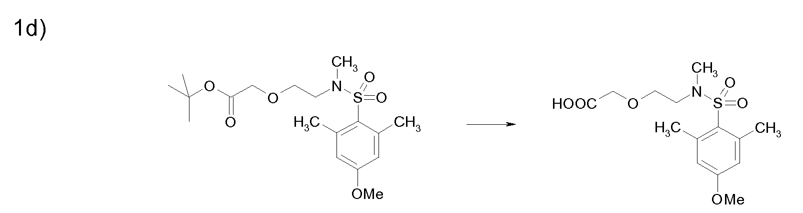

Eine Mischung aus 6.80 g (17.6 mmol) Produkt aus 1c, 8 ml TFA und 100 ml Dichlormethan wird 2.5 Stunden bei Raumtemperatur gerührt. Die Reaktionsmischung wird danach im Vakuum bis zur Trockene eingeengt. Man versetzt den Rückstand mit 1 N Natronlauge und extrahiert zweimal mit Ethylacetat (organische Extrakte verwerfen). Die wässrige Phase wird mit 2 M Salzsäure angesäuert, dann nochmals mit Ethylacetat extrahiert. Die organischen Extrakte werden mit Wasser gewaschen, über Natriumsulfat getrocknet und im Vakuum bis zur Trockene eingeengt.
C₁₄H₂₁NO₆S (331.29)
[M+H]+=332
HPLC (Methode 1): Retentionszeit = 3.4 min

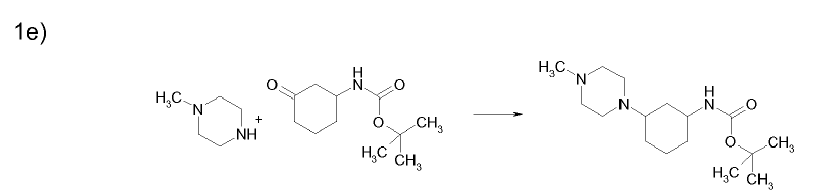

2.6 ml (23.4 mmol) 1-Methylpiperazin, 1.0 g (4.69 mmol) 3-Amino-N-tert-butyloxycarbonylcyclohexanon (AB Chem) und 2.7 ml (49 mmol) Eisessig werden in 10 ml Methanol gelöst und 30 Minuten bei Raumtemperatur gerührt. Danach gibt man portionsweise 1.99 g (9.38 mmol) Natriumtriacetoxyborhydrid zu und rührt 2 Stunden bei Raumtemperatur. Anschließend wird die Reaktionslösung mit Hydrogencarbonatlösung versetzt und mit Dichlormethan extrahiert. Die organische Phase wird im Vakuum vom Lösungsmittel befreit und der Rückstand an RP-Phase (Varian C18 XRS) chromatographiert (Wasser + 5 % NH₃ / Acetonitril = 90:10 -> 0:100).
C₁₆H₃₁N₃O₂ (297.44)
[M+H]+=298

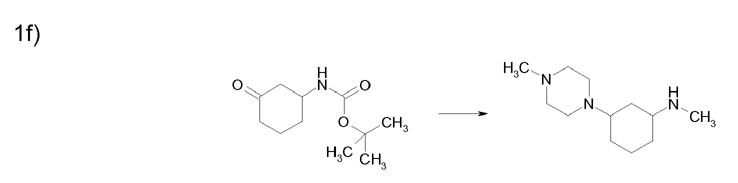

8.57 ml (8.57 mmol) einer 1 M Lösung von Lithiumaluminiumhydrid in Toluol werden in 8 ml THF gelöst und bei Raumtemperatur langsam mit 850 mg (2.86 mmol) Produkt 1e gelöst in 2 ml THF versetzt. Die Reaktionslösung wird 2 Stunden bei 75°C gerührt. Anschließend gibt man 1 N Natronlauge zu und Wasser. Der Niederschlag wird abgesaugt und die Reaktionslösung bis zur Trockene eingeengt.
C₁₂H₂₅N₃ (211.35)
[M+H]+=212
HPLC (Methode 2): Retentionszeit = 0.29 min

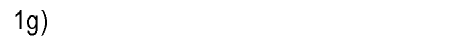

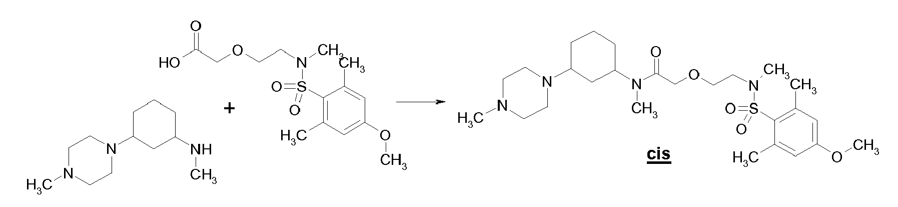

360 mg (1.01 mmol) Produkt 1d (Carbonsäure), 384 mg (1.20 mmol) TBTU und 151 µl (1.09 mmol) Triethylamin werden in 5 ml DMF gelöst und 5 Minuten bei Raumtemperatur gerührt. Dann werden 460 mg (2.18 mmol) Produkt 1f (Amin) zugegeben. Man rührt 2 Stunden bei Raumtemperatur und entfernt anschließend das Lösungsmittel im Vakuum. Zur Trennung des cis/trans-Gemisches wird der Rückstand an RP-Phase (Merck Chromolith Speed ROD) chromatographiert (Wasser + 0.1% Ameisensäure / Acetonitril + 0. 1 % Ameisensäure = 90:10 -> 0:100).Cis-Diastereomer:
C₂₆H₄₄N₄O₅S (524.72)
[M+H]+=525
HPLC (Methode 2): Retentionszeit = 1.53 min

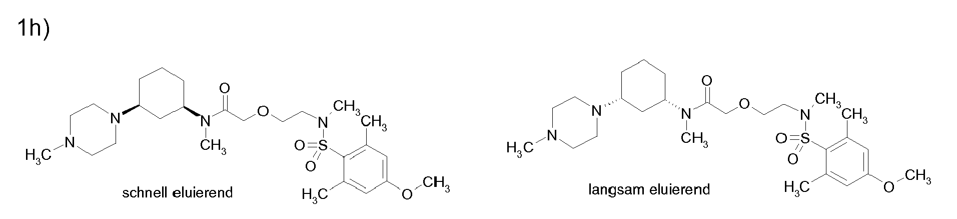

31 mg Produkt 1g (cis-Diastereomer) werden nach HPLC-Methode 3 an chiraler Phase in die Enantiomere aufgetrennt.
HPLC (Methode 3): Retentionszeiten = 7.3 min (schnell eluierendes Enantiomer), 9.7 min (langsam eluierendes Enantiomer)
Analog wurden die folgenden Verbindungen hergestellt:

### Beispiel 2

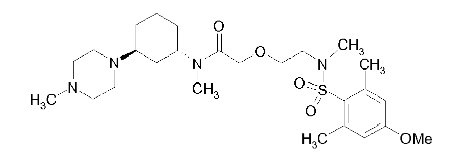

C₂₆H₄₄N₄O₅SxC₂HF₃O₂ (638.74)
[M+H]+=525
HPLC (Methode 6): Retentionszeit = 1.41 min

### Beispiel 3

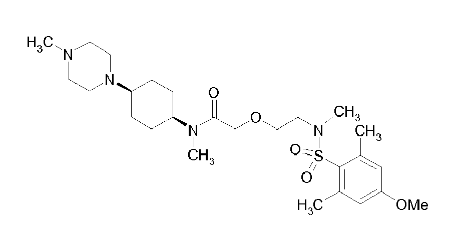

C₂₆H₄₄N₄O₅S x 2C₂HF₃O₂ (752.76)
[M+H]=525
HPLC (Methode 9): Retentionszeit = 1.41 min

### Beispiel 4

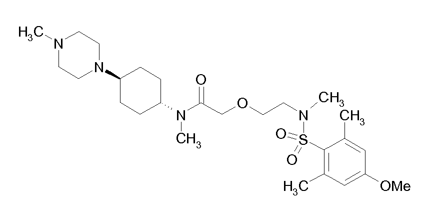

C₂₆H₄₄N₄O₅S (524.72)
[M+H]+=525
HPLC (Methode 9): Retentionszeit = 1.32 min

### Beispiel 5

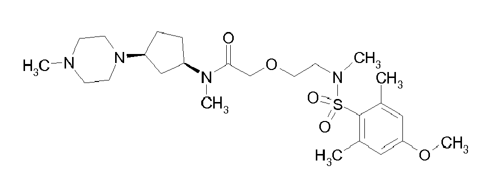

### Beispiel 6

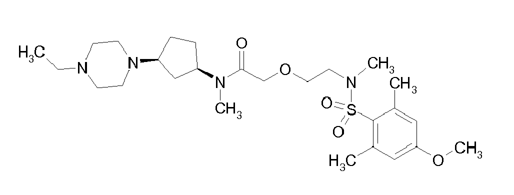

### Beispiel 7

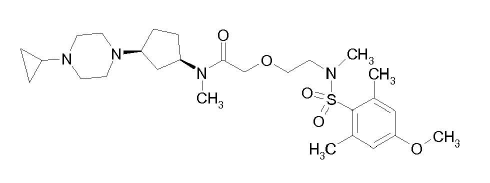

### Beispiel 8

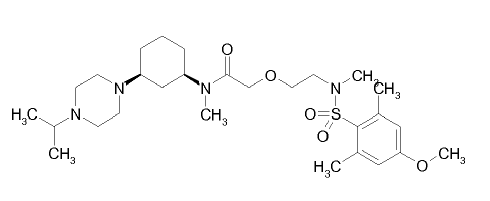

### Beispiel 9

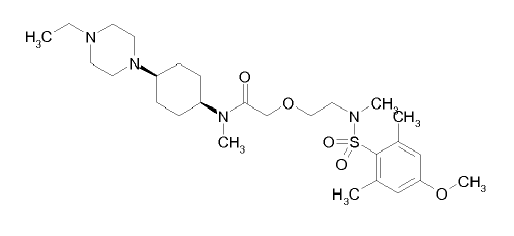

### Beispiel 10

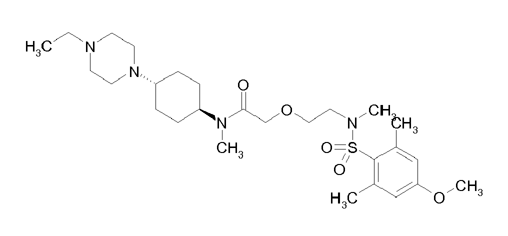

### Beispiel 11

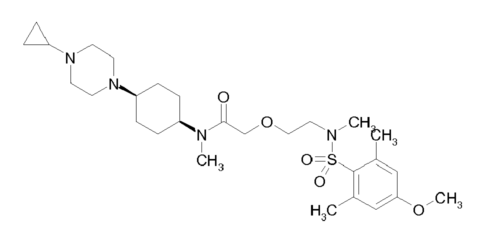

### Beispiel 12

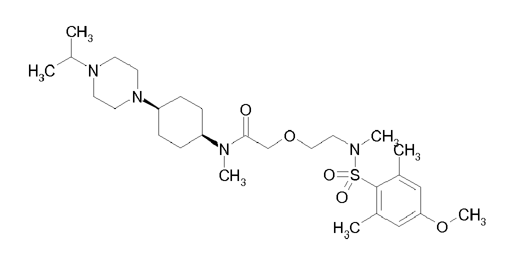

### Beispiel 13

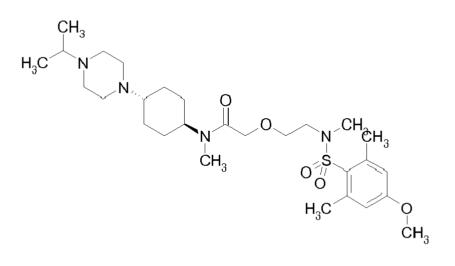

### Beispiel 14

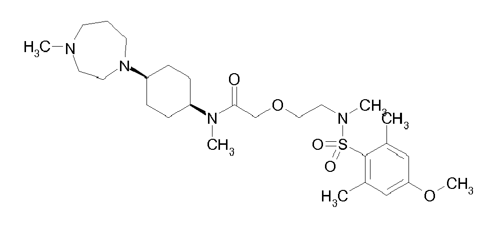

### Beispiel 15

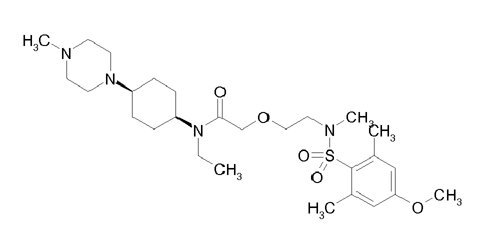

### Beispiel 16

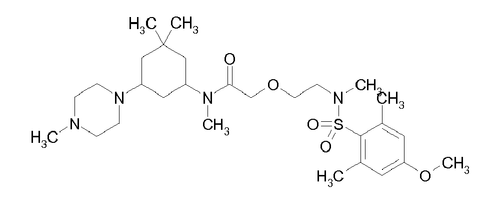

### Beispiel 17

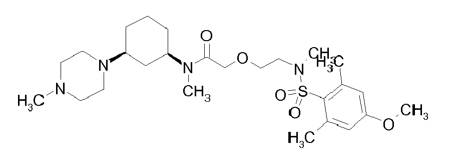

### Beispiel 18

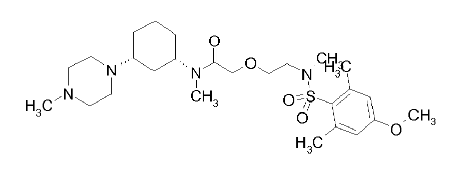

### Beispiel 19

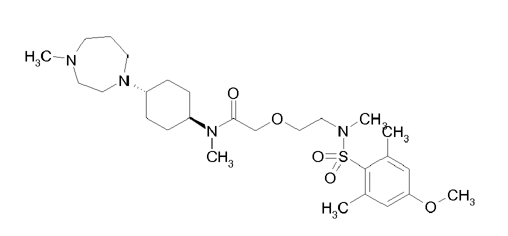

### Beispiel 20

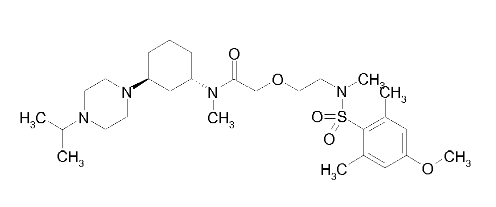

### Beispiel 21

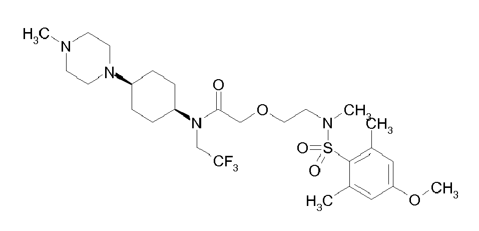

### Beispiel 22

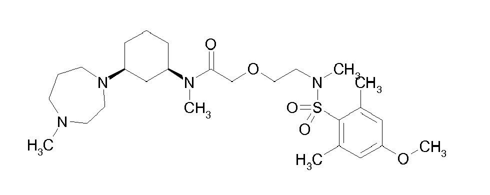

### Beispiel 23

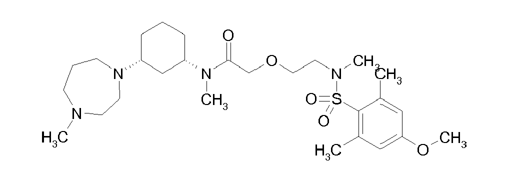

### Beispiel 24

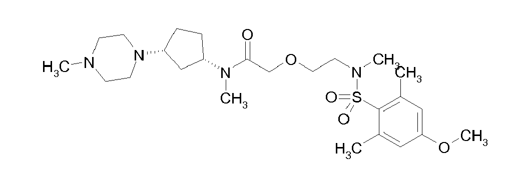

### Beispiel 25

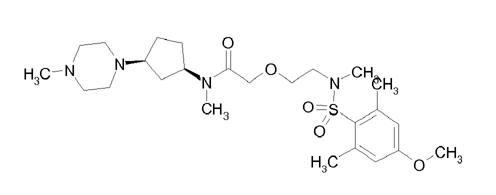

### Beispiel 26

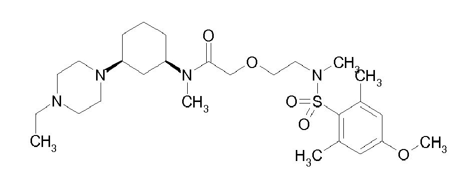

### Beispiel 27

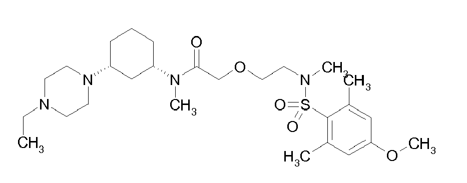

### Beispiel 28

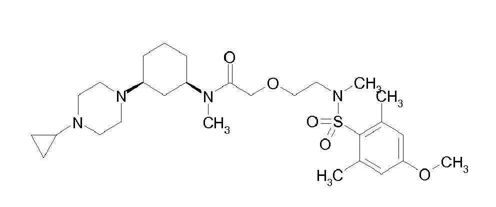

### Beispiel 29

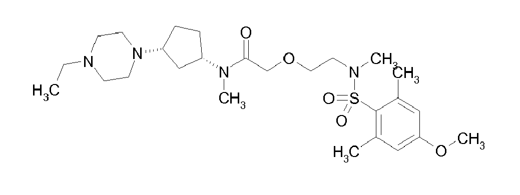

### Beispiel 30

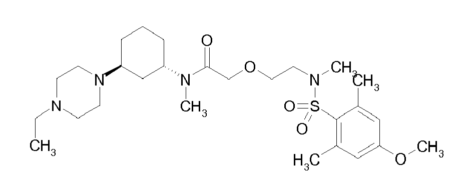

### Beispiel 31

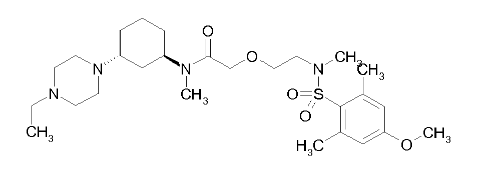

### Beispiel 32

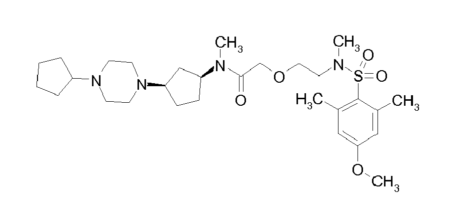

### Beispiel 33

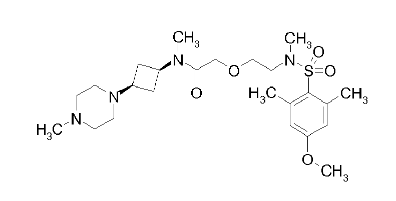

Die nachfolgenden Beispiele beschreiben pharmazeutischer Darreichungsformen, die als Wirkstoff eine beliebige Verbindung der allgemeinen Formel I enthalten:

### Beispiel I

Trockenampulle mit 75 mg Wirkstoff pro 10 ml

### Zusammensetzung:

| | |
|---|---|
| Wirkstoff | 75.0 mg |
| Mannitol | 500 mg |
| Wasser für Injektionszwecke ad | 10.0 ml |

### Herstellung:

Wirkstoff und Mannitol werden in Wasser gelöst. Nach Abfüllung wird gefriergetrocknet. Die Auflösung zur gebrauchsfertigen Lösung erfolgt mit Wasser für Injektionszwecke.

### Beispiel II

Tablette mit 50 mg Wirkstoff

### Zusammensetzung:

| | |
|---|---|
| (1) Wirkstoff | 50.0 mg |
| (2) Milchzucker | 98.0 mg |
| (3) Maisstärke | 50.0 mg |
| (4) Polyvinylpyrrolidon | 15.0 mg |
| (5) Magnesiumstearat | 2.0 mg |
| | 215,0 mg |

### Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe.
   Durchmesser der Tabletten: 9 mm.

### Beispiel III

Tablette mit 350 mg Wirkstoff

### Zusammensetzung:

| | |
|---|---|
| (1) Wirkstoff | 350.0 mg |
| (2) Milchzucker | 136.0 mg |
| (3) Maisstärke | 80.0 mg |
| (4) Polyvinylpyrrolidon | 30.0 mg |
| (5) Magnesiumstearat | 4.0 mg |
| | 600.0 mg |

### Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe.
   Durchmesser der Tabletten: 12 mm.

### Beispiel IV

Kapseln mit 50 mg Wirkstoff

### Zusammensetzung:

| | |
|---|---|
| (1) Wirkstoff | 50.0 mg |
| (2) Maisstärke getrocknet | 58.0 mg |
| (3) Milchzucker pulverisiert | 50.0 mg |
| (4) Magnesiumstearat | 2.0 mg |
| | 160.0 mg |

### Herstellung:

(1) wird mit (3) verrieben. Diese Verreibung wird der Mischung aus (2) und (4) unter intensiver Mischung zugegeben.
   Diese Pulvermischung wird auf einer Kapselabfüllmaschine in Hartgelatine-Steckkapseln Größe 3 abgefüllt.

### Beispiel V

Kapseln mit 350 mg Wirkstoff

### Zusammensetzung:

| | |
|---|---|
| (1) Wirkstoff | 350.0 mg |
| (2) Maisstärke getrocknet | 46.0 mg |
| (3) Milchzucker pulverisiert | 30.0 mg |
| (4) Magnesiumstearat | 4.0 mg |
| | 430.0 mg |

### Herstellung:

(1) wird mit (3) verrieben. Diese Verreibung wird der Mischung aus (2) und (4) unter intensiver Mischung zugegeben.
   Diese Pulvermischung wird auf einer Kapselabfüllmaschine in Hartgelatine-Steckkapseln Gr6Be 0 abgefüllt.

### Beispiel VI

### Suppositorien mit 100 mg Wirkstoff

### 1 Zäpfchen enthält:

| | |
|---|---|
| Wirkstoff | 100.0 mg |
| Polyethylenglykol (M.G. 1500) | 600.0 mg |
| Polyethylenglykol (M.G. 6000) | 460.0 mg |
| Polyethylensorbitanmonostearat | 840.0 mg |
| | 2000.0 mg |

## Patentansprüche

1. Verbindungen der allgemeinen Formel **I** 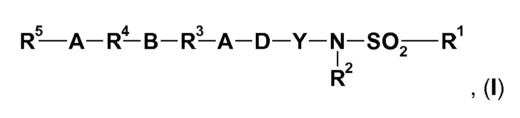 in der
**A** eine Bindung,
**B** eine Bindung,
**D-Y** zusammen eine Gruppe ausgewählt aus 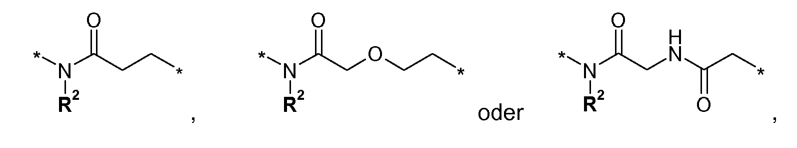
**R¹** die Gruppe 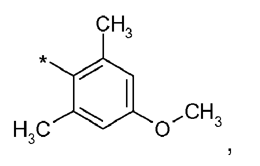
**R²** H oder C₁₋₃-Alkyl-, wobei jede Methylengruppe mit bis zu zwei und jede
Methylgruppe mit bis zu drei Fluoratomen substituiert sein kann, oder auch H₃C-C(O)-,
**R³** eine C₄₋₆-Cycloalkylengruppe, die durch einen, zwei oder drei Reste **R^{3.1}** substituiert sein kann,
**R^{3.1}** -CH₃, -C₂H₅, *iso*-Propyl, *tert*-Butyl, -OH, F, Cl, Br, I,
**R⁴** einen gesättigten 6- oder 7-gliedrigen Diaza-Heterocyclus,
**R⁵** C₁₋₃-Alkyl- oder C₃₋₅-Cycloalkyl bedeutet,
deren Enantiomere, deren Diastereomere, deren Gemische und deren Salze, insbesondere deren physiologisch verträgliche Salze mit organischen oder anorganischen Säuren oder Basen.

2. Verbindungen der allgemeinen Formel **I** gemäß Anspruch 1, in denen
**A** eine Bindung,
**B** eine Bindung,
**D-Y** zusammen eine Gruppe ausgewählt aus 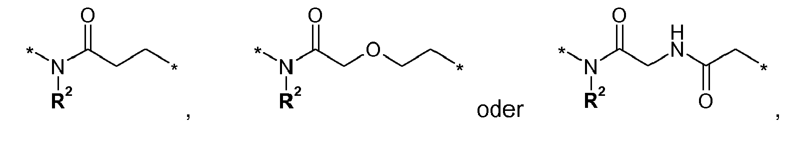
**R¹** die Gruppe 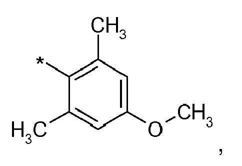
**R²** H oder C₁₋₃-Alkyl-, wobei jede Methylengruppe mit bis zu zwei und jede
Methylgruppe mit bis zu drei Fluoratomen substituiert sein kann, oder auch H₃C-C(O)-,
**R³** eine C₄₋₆-Cycloalkylengruppe,
**R⁴** einen gesättigten 6- oder 7-gliedrigen Diaza-Heterocyclus,
**R⁵** C₁₋₃-Alkyl- oder C₃₋₅-Cycloalkyl bedeutet,
deren Enantiomere, deren Diastereomere, deren Gemische und deren Salze, insbesondere deren physiologisch verträgliche Salze mit organischen oder anorganischen Säuren oder Basen.

3. Verbindungen der allgemeinen Formel **I** gemäß Anspruch 1, in denen **A, B, D, Y, R¹, R², R⁴** und **R⁵** wie in Anspruch 1 definiert sind und
**R³** eine C₄₋₆-Cycloalkylengruppe, die durch einen, zwei oder drei Reste **R^{3.1}** substituiert sein kann, und
**R^{3.1}** -CH₃, -C₂H₅, *iso*-Propyl, *tert*-Butyl, -OH, F, Cl, Br oder **I** bedeutet,
mit der Maßgabe, dass die voranstehend erwähnte C₄₋₆-Cycloalkylengruppe in 1,3-Stellung mit dem restlichen Molekül verknpüft ist, deren Enantiomere, deren Diastereomere, deren Gemische und deren Salze, insbesondere deren physiologisch verträgliche Salze mit organischen oder anorganischen Säuren oder Basen.

4. Verbindungen der allgemeinen Formel **I** gemäß Anspruch 1, in denen **A, B, D, Y, R¹, R², R⁴** und **R⁵** wie in Anspruch 2 definiert sind und
**R³** eine C₄₋₆-Cycloalkylengruppe, die durch einen, zwei oder drei Reste **R^{3.1}** substituiert sein kann, und
**R^{3.1}** -CH₃, -C₂H₅, *iso*-Propyl, *tert*-Butyl, -OH, F, Cl, Br oder I bedeutet,
mit der Maßgabe, dass die voranstehend erwähnte C₄₋₆-Cycloalkylengruppe in 1,3-Stellung mit dem restlichen Molekül verknpüft ist, deren Enantiomere, deren Diastereomere, deren Gemische und deren Salze, insbesondere deren physiologisch verträgliche Salze mit organischen oder anorganischen Säuren oder Basen.

5. Folgende Verbindungen der allgemeinen Formel **I** gemäß Anspruch 1:
| **Nr.** | **Struktur** |
|---|---|
| (1) | 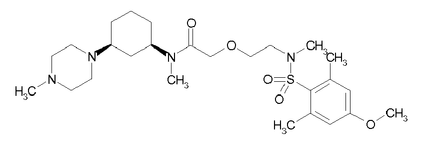 |
| (2) | 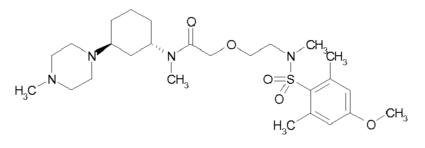 |
| (3) | 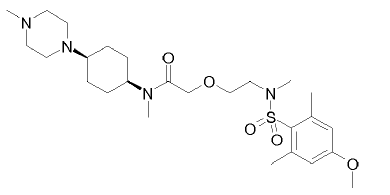 |
| (4) | 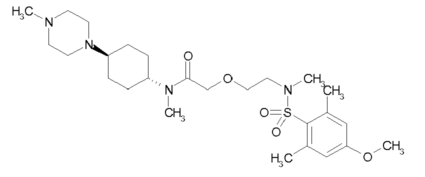 |
| (5) | 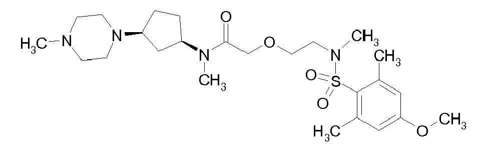 |
| (6) | 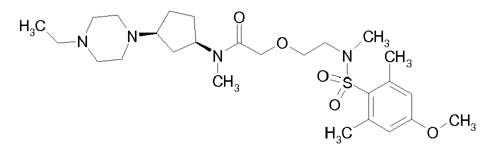 |
| (7) | 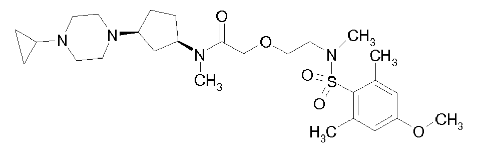 |
| (8) | 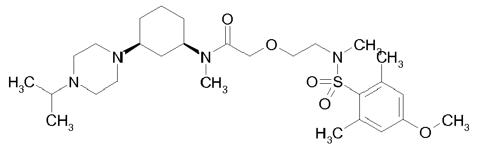 |
| (9) | 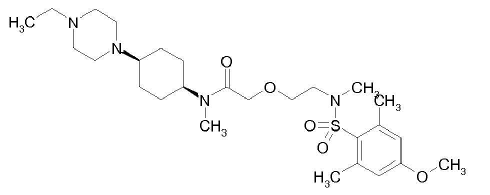 |
| (10) | 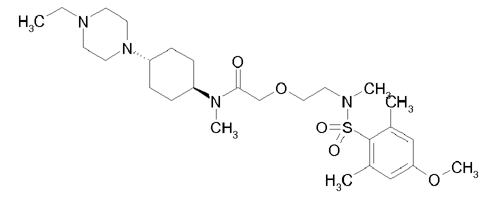 |
| (11) | 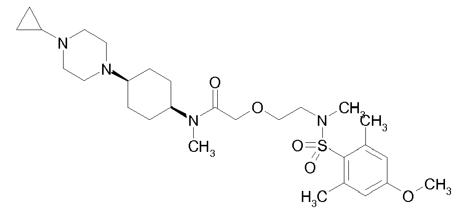 |
| (12) | 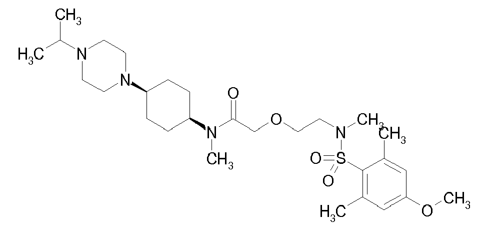 |
| (13) | 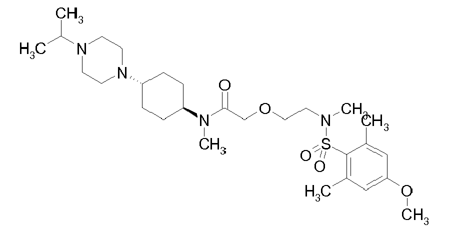 |
| (14) | 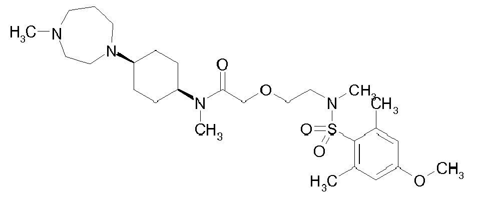 |
| (15) | 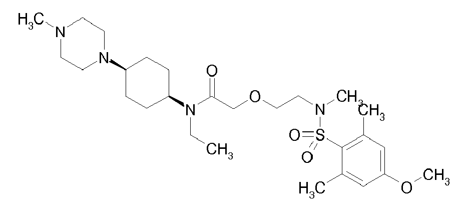 |
| (16) | 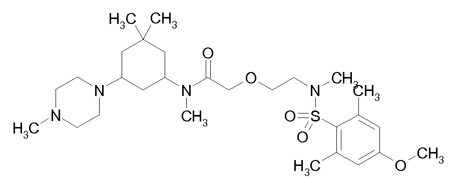 |
| (17) | 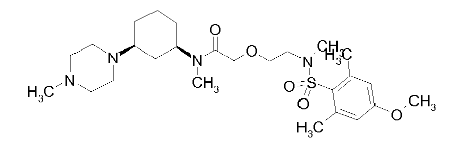 |
| (18) | 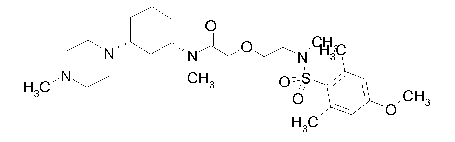 |
| (19) | 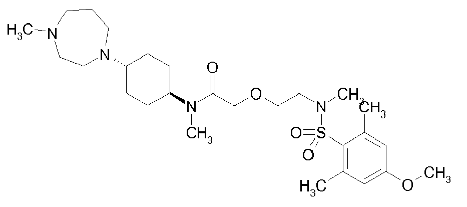 |
| (20) | 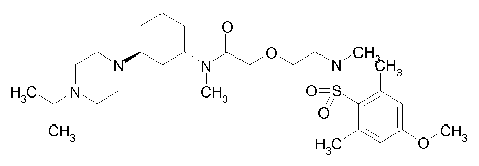 |
| (21) | 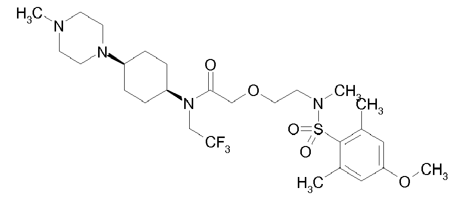 |
| (22) | 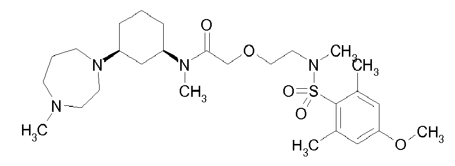 |
| (23) | 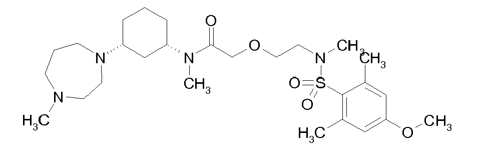 |
| (24) | 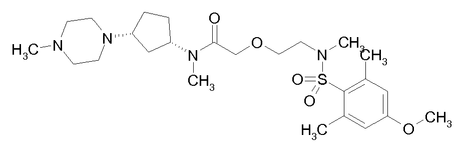 |
| (25) | 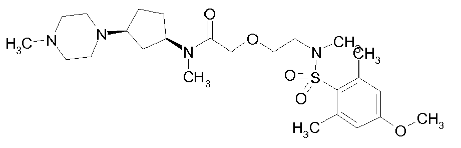 |
| (26) | 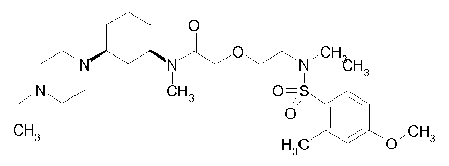 |
| (27) | 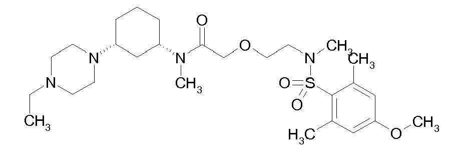 |
| (28) | 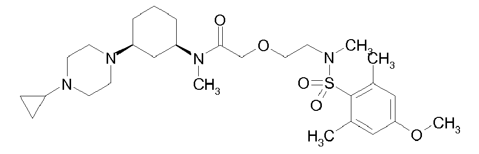 |
| (29) | 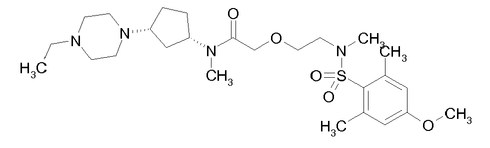 |
| (30) | 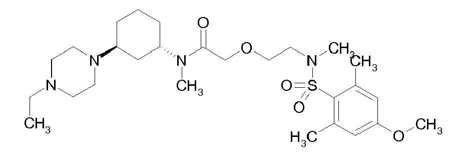 |
| (31) | 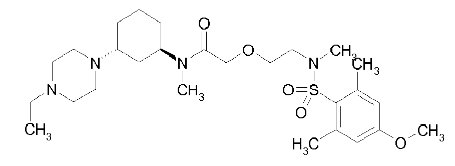 |
| (32) | 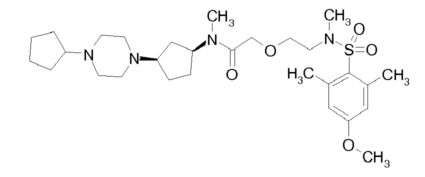 |
| (33) | 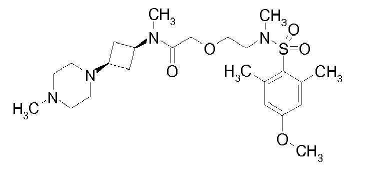 |
deren Enantiomere, deren Diastereomere, deren Gemische und deren Salze, insbesondere deren physiologisch verträgliche Salze mit organischen oder anorganischen Säuren oder Basen.

6. Physiologisch verträgliche Salze der Verbindungen nach einem der Ansprüche 1 bis 5 mit anorganischen oder organischen Säuren oder Basen.

7. Arzneimittel, enthaltend eine Verbindung nach mindestens einem der Ansprüche 1 bis 5 oder ein physiologisch verträgliches Salz gemäß Anspruch 6 neben gegebenenfalls einem oder mehreren inerten Trägerstoffen und/oder Verdünnungsmitteln.

8. Verwendung einer Verbindung nach mindestens einem der Ansprüche 1 bis 6 zur Herstellung eines Arzneimittels zur akuten und prophylaktischen Behandlung von akuten Schmerzen, Eingeweideschmerzen, neuropathischen Schmerzen, entzündlichen / Schmerzrezeptor-vermittelten Schmerzen, Tumorschmerzen und Kopfschmerz-Erkrankungen.

9. Verfahren zur Herstellung eines Arzneimittels gemäß Anspruch 7, **dadurch gekennzeichnet, dass** auf nichtchemischem Weg eine Verbindung nach mindestens einem der Ansprüche 1 bis 6 in einen oder mehrere inerte Trägerstoffe und/oder Verdünnungsmittel eingearbeitet wird.
